# EUROPEAN PATENT SPECIFICATION

(11) **EP 3 677 910 B1**
(45) Date of publication and mention of the grant of the patent: **17.08.2022**
(21) Application number: 18853712.0
(22) Date of filing: 05.09.2018
(51) Int. Cl.: G01N 33/574, G01N 33/573, G01N 33/483, G01N 1/30

(54) **DIAGNOSING PANCREATIC CANCER USING METHIONYL-TRNA SYNTHETASE AND CK19**
DIAGNOSE VON BAUCHSPEICHELDRÜSENKREBS UNTER VERWENDUNG VON METHIONYL-TRNA SYNTHETASE UND CK19
DIAGNOSTIC DU CANCER DU PANCRÉAS À L'AIDE DE MÉTHIONYL-ARNT SYNTHÉTASE ET CK19

(30) Priority: 05.09.2017 KR 20170113255
(43) Date of publication of application: 08.07.2020
(73) Proprietor: Oncotag Diagnostics Co., Ltd., Suwon-si, Gyeonggi-do 16229 (KR)
(72) Inventor: KIM, Sunghoon, Seoul 08826 (KR); KWON, Nam Hoon, Suwon-si Gyeonggi-do 16229 (KR); LEE, Dong Ki, Seoul 03722 (KR); LIM, Beom Jin, Seoul 03722 (KR); JANG, Sung Ill, Seoul 03722 (KR)
(74) Representative: Richardt Patentanwälte PartG mbB
(86) International application number: PCT/KR2018/010369
(87) International publication number: WO 2019/050275

(56) References cited:
- KR-A- 20120 132 592
- US-A1- 2012 264 634
- DATABASE Biosis [Online] 1 April 2016 (2016-04-01), Baek Yi-Yong ET AL: "Methionyl-tRNAsynthetase Serves as a New Prognostic Marker in Pancreatic Cancer", XP055782766, Database accession no. PREV201700234915
- ZAPATA MAURICIO ET AL: "Immunohistochemical expression of SMAD4, CK19, and CA19-9 in fine needle aspiration samples of pancreatic adenocarcinoma: Utility and potential role", CYTOJOURNAL, BIOMED CENTRAL, LONDON, GB, vol. 4, no. 1, 22 June 2007 (2007-06-22), page 13, XP021030338, ISSN: 1742-6413, DOI: 10.1186/1742-6413-4-13
- HUANG KAIJUN ET AL: "Global profiling of the signaling network of papillary thyroid carcinoma", LIFE SCIENCE, PERGAMON PRESS, OXFORD, GB, vol. 147, 9 January 2016 (2016-01-09), pages 9-14, XP029422225, ISSN: 0024-3205, DOI: 10.1016/J.LFS.2016.01.005
- JANG SUNG ILL ET AL: "Sa1449 A NEW STAINING METHOD USING METHIONY-TRNA SYNTHETASE 1 ANTIBODY AND CYTOKERATIN 19 FOR EUS-FNA CYTOLOGY DIFFERENTIATES INDETERMINATE PANCREATIC MASS", GASTROINTESTINAL ENDOSCOPY, ELSEVIER, NL, vol. 91, no. 6, 1 June 2020 (2020-06-01), XP086160753, ISSN: 0016-5107, DOI: 10.1016/J.GIE.2020.03.1245 [retrieved on 2020-06-01]
- MITCHELL, M. L.: "Cytologic Criteria for the Diagnosis of Pancreatic Carcinoma", American Journal of Clinical Pathology, vol. 171, 1 February 1985 (1985-02-01), pages 174-175, XP055581574,
- DATABASE GenBank 23 April 2017 (2017-04-23), "methionine--tRNA ligase, cytoplasmic [Homo sapiens", XP055581581, Database accession no. NP_004981.2
- KARANTZA, V.: "Keratins in health and cancer: more than mere epithelial cell markers", Oncogene, vol. 30, no. 2, 2011, pages 127-138, XP055581585,

## Description

### Technical Field

The present invention relates to a method, use of a composition, and use of a kit as defined in the appended claims for diagnosis of pancreatic cancer.

### Background Art

Cancer collectively refers to a group of diseases, which mainly begin with uncontrollable cell proliferation, invade and destroy adjacent normal tissues or organs, and then create new growing sites therein, thereby finally taking the lives of individuals. Although notable progress has been made over the past decade in regulating controlling cell cycles or apoptosis and seeking new targets encompassing carcinogenic genes or cancer-suppressing genes in order to conquer cancer, the incidence of cancer has increased as civilization has advanced.

Out of these, pancreatic cancer is a fatal cancer that has a 5-year survival rate of 1-4 % and a median survival period of 5 months, and has an extremely poor prognosis as compared with other human cancers. Pancreatic cancer has a poor prognosis since 80-90% of pancreatic cancer patients are diagnosed in a condition where radical resection of pancreatic cancer for expecting complete recovery is not possible, and moreover, the treatment for pancreatic cancer depends mainly on chemotherapy. Therefore, the development of early diagnosis techniques of pancreatic cancer is urgently requested more than any human cancer. The therapeutic effects of several chemotherapeutic agents, including 5-fluorouracil, gemcitabine, and tarceva, which are known to be effective against pancreatic cancer until today, are extremely disappointing, and the rate of response to chemotherapy is only about 15%. These facts suggest that more accurate and prompt diagnosis techniques are required in order to improve the prognosis of pancreatic cancer patients.

The term "pathological examination" refers to an examination that attempts to elucidate the origin of a disease mainly from a morphological point of view by using extracted cells, tissues, or organs. The pathological examination is an important type of examination applied to the diagnosis of a disease by understanding of macroscopic findings, optical microscopy, electron microscopy, or the like. Such pathological examination includes pathologic histology and pathologic cytology. Meanwhile, biopsy and cytodiagnosis have many differences therebetween, and it has been known that biopsy and cytodiagnosis show many differences in terms of prognostic accuracy, such as diagnostic sensitivity and specificity, in assays using well-known cancer markers. Therefore, it is not sure whether a conventionally known cancer marker can substantially provide effective diagnosis for a specific specimen (tissue or cells).

A difficulty in identifying atypical cells is also one of the obstacles in pathological examination of cells isolated from the body. Since Melamed et al., suggested, as squamous cell atypia, a cell change which is not an inflammatory change but is insufficient to diagnose dysplasia, in 1976, there has been much controversy over the diagnosis, interpretation, and treatment strategy of atypical cells. For the resolution of the controversy, The Bethesda System (TBS) was established. TBS extremely restricts the use of the term "atypical cells" so that the term can be used in only the cases where the cell change cannot be diagnosed to be inflammatory, premalignant, or tumoral, that is, of undetermined significance. Therapeutic strategies for atypical cells are difficult since there may be different views therefor. In particular, there is a problem in that in most cases, a diagnosis result of atypical cells is made in examinations at the tissue or cellular level or a diagnosis result of cancer is made in only tissue specimens although cancer is actually progressing. When it is not clearly discriminated whether the indeterminate tissue structure or cell morphology corresponds to an inflammatory lesion or a neoplasm, it is frequently diagnosed as atypism or cellular atypia. Therefore, several repeated re-examinations by another examination measure or the like are needed, resulting in the consumption of significant time and economic costs.

Since the pancreas is located deep in the body, cancer is difficult to detect when the cancer occurs in the pancreas. In cases of pancreatic cancer that is operable through imaging examinations, a definitive diagnosis of pancreatic cancer is needed prior to surgery through biopsy of mass lesions or cytodiagnosis under endoscopic ultrasound. In addition, in cases of inoperable pancreatic cancer, a biopsy or cytodiagnosis is needed for a histological diagnosis for anticancer therapy or radiotherapy. The representative tumor markers for diagnosing pancreatic cancer are CEA (reference value: 5.0 ng/mL) and CA 19-9 (reference value: 37 U/mL). However, CA19-9 has a problem of low specificity as a pancreatic cancer diagnostic marker. A report says that about 1% of people who had health checkups show an increase in CA19-9 level and only 2% of those showing an increase in CA19-9 level without symptoms were actually found to have cancer. The American Cancer Society guidelines have determined that CA19-9 is less sensitive or specific in screening for pancreatic cancer.

Besides, the development of biomarkers for diagnosing pancreatic cancer is actively ongoing. For example, Korean Patent No. 10-0819122 discloses using matrilin, transthyretin, and stratifin as pancreatic cancer markers, and Korean Patent Publication No. 2012-0082372 discloses using several pancreatic cancer markers. In addition, Korean Patent Publication No. 2009-0003308 discloses a method for diagnosing pancreatic cancer by detecting the expression level of REG4 protein in a blood sample of an individual; Korean Patent Publication No. 2012-0009781 discloses an analysis method for measuring the expression level of XIST RNA in cancer tissues isolated from an individual to provide information necessary for diagnosis of pancreatic cancer of the individual; Korean Patent Publication No. 2007-0119250 discloses a novel gene LBFL313 family that is differently expressed in human pancreatic cancer tissue compared with normal human pancreatic tissue; and US Patent Publication No. 2011/0294136 discloses a method for diagnosis of pancreatic cancer using biomarkers, such as keratin 8 protein.

However, the above-mentioned markers have a limitation in that the markers show a large difference in diagnostic efficiency and accuracy thereof, and are not particularly based on cytological analysis. As for atypical cells that cannot be clearly discriminated whether they are clinically tumor cells or in other disease states, an accurate diagnosis of whether the atypical cells correspond to pancreatic cancer is more important, but there is no marker for accurate diagnosis. Conventionally reported diagnostic markers for pancreatic cancer fail to attain effective diagnosis when applied to cytodiagnosis since such diagnostic markers had poor sensitivity and specificity in cell-level diagnosis unlike when applied to biopsy.

Therefore, in order to diagnose pancreatic cancer, high-priced thorough examinations, such as computed tomography (CT), endoscopic retrograde cholangiopancreatography (ERCP), endoscopic ultrasound (EUS), and angiography, are required in addition to the tumor markers. However, even through these examinations, an accurate diagnosis of pancreatic cancer is very difficult. Moreover, most cases of pancreatic cancer may be advanced cancer cases that have already been unresectable at the time of diagnosis, and only 10-15% of pancreatic cancer cases are operable. A diagnosis of pancreatic cancer is made on inoperable patients through endoscopic ultrasound fine needle aspiration examinations. However, cytodiagnosis through endoscopic ultrasound fine needle aspiration examinations has a limit in definitive diagnosis since the obtained cells cannot be compared with surrounding cells or structures unlike postoperative pancreatic tissues where surrounding structures are intact.

For this reason, the discrimination between pancreatic cancer cells and cells of other diseases (e.g., pancreatitis) still mainly depends on pathological diagnosis based on general staining, such as H&E staining or pap staining. However, the definitive diagnosis of pancreatic cancer by conventional staining may cause different diagnoses according to the experience and interpretation of medical staffs, and especially when the pancreatic cells are identified as atypical cells by pancreatic cell observation through H&E staining or pap staining, it is difficult to discriminate whether the pancreatic cells correspond to pancreatic cancer or a benign disease, and as a result, accurate diagnosis and treatment with respect to diseases of patients are not performed promptly. Since the confirmation on cytodiagnosis is not accurate, appropriate treatment cannot be performed on operable pancreatic cancer patients, and conversely, unnecessary surgery is difficult to prevent. Therefore, to clinically enhance the treatment effect of pancreatic cancer, an accurate diagnosis with respect to cytodiagnosis is needed.

MRS has been employed in pancreatic cancer prognosis. See Database Biosis [Online] 1 April 2016 (2016-04-01), Baek Yi-Yong et al: "Methionyl-tRNA synthetase serves as a New Prognostic Marker in Pancreatic Cancer", XP55782766, Database accession no. PREV201700234915. See also, US 2012/264634A1 to Amersdorfer. However, the cited prior art does not disclose the additional use of the CT19 marker which results in significantly improved diagnostic accuracy.

### Detailed Description of the Invention

### Technical Problem

The present inventors performed a careful cytological analysis using pancreatic cell samples obtained from pancreatic cancer clinical patients in order to find markers capable of more accurately diagnosing pancreatic cancer at the cellular level, and as a result, the present inventors first established that malignant tumor cells can be clearly discriminated through the detection of (high) expression of methionyl-tRNA synthetase (MRS) in pancreatic cell samples, and especially, such a discrimination can be made on even cell samples that are identified as atypical cells by conventional pathologic cytology using H&E staining or pap staining and thus cannot be definitively diagnosed for tumor or non-tumor, and completed the present invention.

### Technical Solution

The solution is provided by a method according to independent claim 1, use of a composition according to independent claim 6 and use of a kit according to independent claim 10. Further embodiments are provided in the dependent claims.

Throughout this disclosure, various aspects or conditions related to the invention can be proposed in a range format. In this specification, the description of a range value encompasses the boundary value unless otherwise stated, in other words, it is meant to encompass all of the values below the lower limit and above the upper limit. It should be understood that the description of the range format is merely for convenience and brevity and should not be construed as an inflexible limitation on the scope of the invention. Accordingly, the description of a range should be considered to have specifically disclosed all the possible subranges as well as individual numerical values within that range. For example, a description of a range such as from 1 to 5 should be considered to have specifically disclosed subranges, such as 1 to 3, 1 to 4, 2 to 5, 2 to 3, 2 to 4, and 3 to 4, as well as individual numbers within that range, for example, 1, 2, 2.7, 3, 3.5, 4.3, and 5. This applies regardless of the breadth of the range.

As used herein, the term "comprising" is used synonymously with "containing" or "being characterized by", and does not exclude additional elements or steps that are not recited in compositions or methods. The term "consisting of" means excluding additional elements, steps, or ingredients that are not separately described. The term "consisting essentially of" means that in the scope of the compositions or methods, the term includes described ingredients or steps as well as any element or step that does not substantially influence basic characteristics of the compositions or methods.

As used herein, the term "diagnosis" refers to identifying (determining) the presence or characteristics of a pathological condition. Specifically, in the present disclosure, the diagnosis may be an identification of the presence or absence of pancreatic cancer by determining the expression or non-expression of an MRS protein or the expression level of the MRS protein.

As used herein, the term "MRS" refers to methionyl-tRNA synthetase, and the MRS is an enzyme that mediates aminoacylation of tRNA with amino acid methionine. As long as the MRS protein of the present disclosure includes an MRS amino acid sequence known in the art, the specific sequence thereof and the biological origin thereof are not particularly limited. For example, MRS is encoded by the MRAS gene in human, and the sequence information of MRS is known by a Genbank accession number, such as NM_004990 (mRNA), NP_004981.2, or P56192.2 (protein). Preferably, the MRS of the present disclosure may comprise an amino acid sequence of a human MRS protein defined by SEQ ID NO: 1. More preferably, the MRS protein of the present disclosure may consist of an amino acid sequence defined by SEQ ID NO: 1. The MRS has two isoforms: a cytoplasmic form (cytoplasmic methionyl-tRNA synthetase); and a mitochondrial form (mitochondrial methionyl-tRNA synthetase). The MRS in the present disclosure may be preferably in a cytoplasmic form.

As used herein, the term "expression" refers to the production of a protein or a nucleic acid in cells.

As used herein, the term "protein" is used interchangeably with the term "polypeptide" or "peptide", and refers to, for example, a polymer of amino acid residues, as is usually found in proteins in nature.

As used herein, the term "pancreatic cancer" refers to a malignant neoplasm that has characteristics of a fast proliferation rate, infiltration into surrounding tissues, and metastasis to other organs, including a malignant tumor or cancer occurring in the pancreas. The malignant tumor or cancer is distinguished from a benign tumor having characteristics of a slow growth rate and non-metastasis.

More than 90% of cancer cases occurring in the pancreas are developed in pancreatic duct cells, and pancreatic cancer usually means pancreatic ductal cancer or pancreatic ductal adenocarcinoma. Therefore, the pancreatic cancer in the present disclosure may preferably mean pancreatic ductal adenocarcinoma.

The pancreatic cancer as a target of diagnosis in the present disclosure is not particularly limited to the cause thereof regardless of whether the pancreatic cancer is a primary cancer, or a cancer secondarily occurring in the pancreas by metastasis. Preferably, a primary cancer may be a target of diagnosis.

As used herein, the term "normal" refers to a state of not being a malignant tumor or cancer (negative for malignancy), and the term is meant to encompass a completely normal state without any disease, another disease state, such as pancreatitis but not a malignant tumor (cancer), or/and a determination state corresponding to "benign". As used herein, a benign indication described as "benign" in the determination of the clinical state of a (final) disease is distinguished from a positive indication denoted by "positive" on a corresponding examination, and the positive indication denoted by "positive" means that there is a response in the corresponding examination or there is a result indicating the probability of cancer in the corresponding examination.

Approximately 5-10% of pancreatic cancer patients have genetic predispositions, and the incidence of pancreatic cancer in the family population with pancreatic cancer family history is about 7.8%, which is higher than the incidence of pancreatic cancer in general population, 0.6%. The pancreatic cancer has a 5-year survival rate of 5% or less, showing an extremely poor prognosis. The reasons are that: surgical resection is possible within 20% of cancer cases since most cases of pancreatic cancer are found after cancer progression; the micro-metastasis results in a little improvement in the survival rate although complete resection is made in view of the naked eye; and pancreatic cancer has a low response to chemotherapy and radiotherapy.

At present, pancreatic cancer is diagnosed by a biopsy using computed tomography (CT) or magnetic resonance imaging (MRI), and the imaging examination CT or MRI is used, but corresponds to an indirect diagnosis method. Ultimately, the diagnosis of pancreatic cancer is carried out through a biopsy or cytodiagnosis corresponding to a pathological method. The biopsy enables a definitive diagnosis of cancer in a particular region through a comparison with surrounding structures or cells, but the cytodiagnosis cannot prove a relationship with surrounding tissues since individual cells are collected and smeared. Therefore, there are fundamentally many differences between biopsy and cytodiagnosis.

In recent years, the presence or probability of pancreatic cancer is determined by carrying out protein assay together in a body fluid, such as plasma, as a clinical sample that can be used for diagnosis of pancreatic cancer. However, clinical serology is merely used as a reference material for diagnosing pancreatic cancer, and do not provide conclusive information in the diagnosis of pancreatic cancer. Currently, an example of a tumor indicator that is most commonly used with respect to pancreatic cancer may be carbohydrate antigen 19-9 (CA19-9) or carcinoembryogenic antigen (CEA). However, CA19-9 is known to be unsuitable for the diagnosis of pancreatic cancer since the level of CA19-9 in the blood rises even in noncancerous diseases, such as hepatitis, cirrhosis, and pancreatitis, and CA19-9 is not specific to pancreatic cancer since CA19-9 is a marker that is detectable in the blood but not pancreatic cells per se. Therefore, CA19-9 is used to determine the prognosis of a patient by monitoring the serum CA19-9 level after surgery. CEA also has a limitation in the accurate diagnosis of pancreatic cancer since CEA fails to show sufficient sensitivity, specificity, positive predictive value and/or negative predictive value, and these facts are well described in examples herein.

On the contrary, the present inventors not only first established that MRS is (highly) expressed in pancreatic cancer, but also established that the use of MRS as a pancreatic cancer marker can lead to diagnostic results with high accuracy in cytodiagnosis as well as biopsy. That is, MRS is specifically highly expressed in pancreatic cancer cells rather than normal pancreatic cells (i.e., non-tumoral pancreatic cells), and MRS has been revealed to show remarkable effects that MRS enables the determination of pancreatic cancer on pancreatic cell samples with higher accuracy than that of CEA, which has been most used as a pancreatic cancer marker, and especially, in cytodiagnosis, enables the discrimination of pancreatic cancer cells with high accuracy even on atypical cells that are difficult for definitive diagnosis by conventional pathologic cytology (e.g., H&E staining or pap staining).

Accordingly, the present invention provides a method according to independent claim 1.

The subject of the present disclosure may be an animal, preferably an animal including a mammal, and especially a human, and includes cells, tissues, organs, and the like derived from the animal. More preferably, the subject may be a human or a patient in need of diagnosis. In the present disclosure, a step of providing the sample from the subject or the latent patient may be performed before step (a).

As used herein, the term "latent patient" refers to a patient suspected of having pancreatic cancer, and means a patient suspected of having pancreatic cancer through various examinations, such as clinical symptoms, hematological examinations, or imaging examinations.

That is, the latent patient includes a patient who can and cannot be diagnosed with pancreatic cancer through imaging examinations, and also means a patient who is suspected of having pancreatic cancer through clinical symptoms or hematological examinations even though the patient cannot be diagnosed with pancreatic cancer through imaging examinations. Examples of the clinical symptoms that pancreatic cancer patients may include abdominal pain, jaundice, weight loss, indigestion, and diabetes, but these symptoms are not specific only to pancreatic cancer. In addition, the levels of jaundice or diabetes or a tumor marker, such as CEA or CA19-9, may be increased in hematological examinations. Imaging examinations, such as abdominal ultrasound, abdominal CT, abdominal MRI, and PET-CT, may be carried out, and in these imaging examinations, the presence of a pancreatic mass raises a suspicion of pancreatic cancer. These imaging examinations can lead to a suspicion of pancreatic cancer, but not a definitive diagnosis of pancreatic cancer. The final definitive diagnosis of pancreatic cancer is carried out by pathological examinations. An operable patient is definitively diagnosed through tissues obtained after surgery, and an inoperable patient is definitely diagnosed mainly through cytodiagnosis.

Preferably, the latent patient (a patient with suspected pancreatic cancer) of the present disclosure may mean a patient having general symptoms, such as abdominal pain, jaundice, weight loss, indigestion, and diabetes, and cannot be definitively diagnosed with pancreatic cancer merely through diagnostic equipment, such as CT, ultrasound, and MRI. More preferably, the latent patient may be a patient who cannot or need not have a wide area of invasive biopsy (i.e., the surgical pancreatic biopsy is impossible or unnecessary) and thus is in need of a clear diagnosis of pancreatic cancer on the basis of cytology (cytodiagnosis). In other words, the latent patient may be a patient in need of clear diagnosis of pancreatic cancer through analysis at the cellular level, but is not limited thereto.

The sample comprises pancreatic cells. The pancreatic tissue may be obtained from any site (especially, a suspected lesion site) of the pancreas including pancreatic ducts. The pancreatic tissue may be generally obtained by biopsy or surgery of the pancreas. The isolation method for the pancreatic cells is not particularly limited, and is understood as a concept encompassing current methods used to isolate cells of human tissues in the art as well as new methods to be developed for the same purpose in the future. The isolation method may be preferably brushing cytology or fine needle aspiration (FNA), and most preferably endoscopic ultrasound fine needle aspiration (EUS-FNA).

As used herein, the term "collected by brush cytology" means a manner in which cells are collected by brushing the pancreas, especially, pancreatic duct surface (especially, a suspected lesion site) using an ordinary cytology brush.

As used herein, the term "isolated by fine needle aspiration" means a collection manner in which the cells of a lesion (a suspected pancreas cancer site) are aspired and extracted using ordinary used fine needle in cytodiagnosis.

Preferably, in an embodiment, the pancreatic tissue or pancreatic cell sample essentially comprises pancreatic duct cells. The pancreatic duct is distinguished from the pancreatic parenchyma.

The obtained pancreatic cells or tissue may be pretreated according to an ordinary sample pretreatment manner (e.g., fixation, centrifugation, smearing on slides, etc.) known in the art.

In one preferable embodiment, the pancreatic cell or tissue sample may be pretreated by an ordinary paraffin block or paraffin section manufacturing method before being provided on test slides.

In one preferable embodiment, the pancreatic cell or tissue sample may be prepared after being pretreated by an ordinary liquid-based monolayer slide manufacturing method (a method for manufacturing a slide for liquid-based cytology). For example, the pancreatic cell or tissue sample may be provided on a test slide by a liquid-based monolayer attachment method using ThinPrep, SurePath, CellPrep, or the like.

The method for diagnosis of pancreatic cancer of the present disclosure may be preferably an analysis of pancreatic cells. Cytological analysis for directly analyzing pancreatic cells has many differences from biopsy, and thus is much different from the pancreatic cancer diagnosis methods reported in the prior art documents described herein. Furthermore, the method of the present disclosure uses pancreatic cells per se, and thus has no likelihood of confusion with tumors of other organs.

In a conventional biopsy, a target site is endoscopically observed, or a tissue with a predetermined area of about 1 g to 10⁹ cells is collected from a tissue suspected of being transformed into cancer, followed by cancer diagnosis through a biochemical manner, such as staining. It is known that such a biopsy comparatively facilitates a definitive diagnosis of cancer present in a particular region through comparison with surrounding structures or cells. The expression patterns of markers at the tissue level also facilitate a definitive diagnosis through the comparison of overall tendencies with surrounding normal tissues. However, cytodiagnosis cannot prove a relationship with surrounding tissues since separate cells are collected and smeared, and thus cytodiagnosis has considerable difficulty in diagnosis. Therefore, the diagnosis of diseases at the cellular level is significant.

As used herein, the term "detection" is meant to encompass all of a measurement and identification of the presence (expression) or absence of a target substance (a marker protein, MRS or/and CK19 in the present disclosure), or a measurement and identification of a change in presence level (expression level) of a target substance. In the same context, measuring the expression level of the protein means measuring the expression or non-expression (that is, measuring the presence or absence of expression) or measuring the level of qualitative or quantitative change of the protein. The measurement may be carried out without limitation, including all of qualitative methods (analyses) and quantitative methods. In the measurement at the protein level, the kinds of qualitative and quantitative methods are well known in the art, and the test methods described herein are included therein. Specific comparisons of the protein level for respective methods are well known in the art. Therefore, the detection of the MRS protein is meant to encompass detecting the presence or absence of the MRS protein or identifying the increase in expression level (up-regulation) of the protein.

As used herein, the term "increase in expression (or high expression)" of a protein means that a previously unexpressed one is expressed (that is, a previously undetected one is detected) or an overexpression is relatively shown relative to a normal level (that is, the detected amount is increased). This may imply performing a step comprising a comparison or contrast with a negative control. It can be understood by a person skilled in the art that the meaning of the term opposite thereto has an opposite meaning on the basis of the definition.

In the present disclosure, the detection of the MRS protein is not particularly limited to a method therefor as long as the detection is carried out by a protein expression level measurement method known in the art, but for example, the detection or measurement may be carried out using an antibody specifically binding to the protein. Specifically, the detection of the protein may be carried out by, for example, any one selected from the group consisting of, but is not limited to, western blotting, enzyme linked immunosorbent assay (ELISA), radioimmunoassay, radioimmunodiffusion, Ouchterlony immunodiffusion, rocket immunoelectrophoresis, immunohistostaining (including immunohistochemical staining, immunocytochemical staining, and immunofluorescence staining), immunoprecipitation assay, complement fixation assay, fluorescence activated cell sorter (FACS) assay, surface plasmon resonance (SPR) assay, or protein chip assay. Besides, in the measurement method is understood in accordance with the descriptions below of an agent for measuring the expression level of MRS and a kit comprising the same, which are provided in the present disclosure.

Specifically, the method for detecting a methionyl-tRNA synthetase (MRS) protein in a pancreatic sample collected from a latent patient in order to provide information necessary for the diagnosis of pancreatic cancer may be performed by measuring the expression or non-expression or expression level of a methionyl-tRNA synthetase protein in a pancreatic sample collected from a latent patient and determining the latent patient to be a pancreatic cancer patient when the methionyl-tRNA synthetase protein is (highly) expressed. The method has an advantage in that pancreatic cancer can be immediately determined with a considerable level of accuracy through the detection of a (high) expression of MRS even without comparison with another control group. This is well described in the examples of the invention.

According to an example of the present disclosure, MRS was strongly (highly) expressed in malignant tumor cells. That is, it was verified that MRS can be used as a pancreatic cancer diagnostic marker with a considerable predetermined level of accuracy.

In particular, it was verified that MRS was highly expressed and detected even in pancreatic cells of a patient wherein the pancreatic cells were identified as atypical cells by conventional cytopathological diagnosis (based on H&E staining, pap staining, or the like) and thus undiagnosable but afterward the patient was finally diagnosed with pancreatic cancer as a result of follow-up observation of the patient. Considering that it is very difficult to discriminate whether atypical cells correspond to a tumor or other benign disease, such as pancreatitis, by conventional cytopathological diagnosis (based on H&E staining, pap staining, or the like) generally used for cancer diagnosis, the determination of whether such atypical cells correspond to a tumor is clinically very important, and it is very meaningful that atypical cells can be determined to be tumor cells when a (high) expression of MRS is observed in the atypical cells.

Previously reported pancreatic cancer diagnostic markers fail to attain effective diagnosis when applied to cytodiagnosis, unlike when applied to biopsy, since such diagnostic markers had poor sensitivity and specificity in the diagnosis at the cellular level. According to another example of the present disclosure, in cases of CEA as one of the diagnostic markers most generally used in the diagnosis of pancreatic cancer, the expression of CEA was not observed in pancreatic cells, or the extent of expression of CEA, if any, was very weak. In addition, CEA was not expressed in cells identified as atypical cells (afterward, finally diagnosed with pancreatic cancer) as a result of H&E staining. In other words, previously reported pancreatic cancer diagnostic markers do not show consistent expression in atypical cells that are not clearly identified as pancreatic cancer cells or normal cells by conventional cytopathological diagnosis using H&E staining or the like, failing to attain a clear diagnosis, but the MRS of the present disclosure can attain a clear diagnosis of whether even pancreatic cells identified to be atypical are tumor cells, leading to a more accurate diagnosis of pancreatic cancer.

Conventionally, for diagnosis of pancreatic cancer, high-priced thorough examinations, such as computed tomography (CT), endoscopic retrograde cholangiopancreatography (ERCP), endoscopic ultrasound (EUS), and angiography, are required, but even through these examinations, an accurate diagnosis of pancreatic cancer is very difficult. Ultimately, a pathological method, such as biopsy or cytodiagnosis, needs to be performed for definitive diagnosis of pancreatic cancer. Meanwhile, most cases of pancreatic cancer may be advanced cancer cases that have already become unresectable at the time of diagnosis, and only 10-15% of pancreatic cancer cases are operable. A diagnosis of pancreatic cancer is carried out for inoperable patients through endoscopic ultrasound fine needle aspiration examinations. However, cytodiagnosis through endoscopic ultrasound fine needle aspiration examinations has a limit in definitive diagnosis since the obtained cells cannot be compared with surrounding cells or structures unlike postoperative pancreatic tissues where surrounding structures are intact.

Conventional cytopathological diagnosis mainly depends on general staining, such as H&E staining or pap staining, to discriminate pancreatic cancer cells from cells of other diseases (e.g., pancreatitis). However, when a definitive diagnosis of pancreatic cancer is made by a conventional cytopathological diagnostic method based on such conventional general staining, different diagnoses are made depending on experiences and analysis techniques of medical staffs, and these conventional examinations often show pathological findings as atypical cells that are not clearly identified as pancreatic cancer cells or normal cells (having a comprehensive meaning of non-pancreatic cancer cells, for example, including pancreatitis cells or the like), and thus requiring a plurality of additional and repetitive re-examinations.

Especially, when pancreatic cells are identified as atypical cells in the pancreatic cell observation through H&E staining or pap staining, it is very difficult to discriminate whether the pancreatic cells correspond to pancreatic cancer or other benign disease, failing to make a fast accurate diagnosis and treatment for a diseased patient. Since the accurate diagnosis in cytodiagnosis is delayed, an appropriate treatment cannot be performed on operable pancreatic cancer patients, and conversely, unnecessary surgery is difficult to prevent. Therefore, to clinically enhance the treatment effect of pancreatic cancer, an accurate diagnosis with respect to cytodiagnosis is needed.

Considering that it is very difficult to discriminate whether atypical cells correspond to a tumor or other benign disease by H&E staining or pap staining that is generally used for cancer diagnosis, the determination of whether such atypical cells correspond to a tumor is clinically very important, and it is very meaningful that atypical cells can be determined to be tumor cells when a (high) expression of MRS is observed in the atypical cells. Furthermore, considering that compared with cytodiagnosis, a biopsy requiring relatively large amounts of biopsy material increases the physical burden on patients in terms of sample acquisition, and in some cases, surgery is impossible and tissue collection is impossible depending on the progression of cancer, the MRS marker of the present disclosure, which provides an accurate diagnosis even at the cellular level, has even greater advantages.

In the present disclosure, a pathological examination based on morphological diagnosis that has been used may be carried out before, simultaneously with, or after the detection of the MRS protein (or the step of measuring the expression level of the MRS protein. That is, steps (i) and (ii) below may be further performed before, simultaneously with, or after the detection of the MRS protein (or the step of measuring the expression level of the MRS protein):
(i) staining the pancreatic cells collected from the latent patient with: at least one staining solution selected from the group consisting of 4',6-diamidino-2-phenylindole (DAPI), methylene blue, acetocarmine, toluidine blue, hematoxylin, and Hoechst, which stain nuclei; and at least one solution selected from the group consisting of eosin, crystal violet, and Orange G, which stain cytoplasm; and
(ii) identifying the pancreatic cells as malignant tumor cells, atypical cells, or normal cells, through the staining of the pancreatic cells.

The atypical cells identified in step (ii) are understood as undiagnosed and un-diagnosable cells, and specifically, the atypical cells may be meant to also encompass, but are not limited to, a determination of being suspicious of malignancy in the pathological examination based on morphological diagnosis.

The steps (i) and (ii) above are involved in the cytodiagnosis method according to a pathological examination based on morphological diagnosis, and follow H&E staining and pap staining used in an example of the present disclosure. As used herein, the term "pathological examination based on morphological diagnosis" or "morphological examination" refers to an examination of an abnormal morphological change when normal cells are transformed into cancer.

Specific examination items or their criteria with respect to the abnormal morphological change are not particularly limited as long as the abnormal morphological change is a kind of morphological change of cancer cells in the art, but at least one selected from the group consisting of: cell clustering; the nuclear to cytoplasmic ratio (N/C ratio); the shape of the nuclear membrane (irregularity of the nuclear membrane shape); chromatin clumping; an appearance of nucleoli in the nucleus; and an appearance of mitosis may be examined. The morphological examination may be performed simultaneously with, separately from, or sequentially with the foregoing method for providing information necessary for the diagnosis of pancreatic cancer.

In step (ii), the identifying of the pancreatic cell sample as malignant tumor cells, atypical cells, or normal cells from the cell-staining results in step (i) may be made on the basis of abnormal morphological changes when normal cells are transformed into cancer, and the specific criteria for identification are well known in the art. The "atypical cells" means cells that cannot be clearly identified as malignant tumor cells (cancer cells) or normal cells by a morphological change.

In one preferable embodiment of the present disclosure, in step (ii), the identifying of the pancreatic cell sample to be malignant tumor cells, atypical cells, or normal cells from the cell-staining results in step (i) may be performed according to the following criteria:
the pancreatic cells are identified as malignant tumor cells when the pancreatic cells show two or more types of morphological abnormality selected from the group consisting of: a three-dimensional smear of cells; a high nuclear to cytoplasmic ratio (N/C ratio); an appearance of chromatin clumping; a rough-shaped nuclear membrane; an appearance of nucleoli, and an appearance of mitosis;
the pancreatic cells are identified as normal cells when the pancreatic cells are smeared in one layer; the nuclear to cytoplasmic ratio (N/C ratio) is small; and the nuclear membrane has a smooth shape; and
the pancreatic cells are identified as atypical cells when the pancreatic cells have neither a cell change leading to malignant tumor cells, nor can be determined to be normal.

When steps (i) and (ii) are further performed in parallel before, simultaneously with, or after the MRS detection (expression level measurement) step, diagnostic results with very high accuracy can be obtained merely through an examination at the cellular level (that is, cytodiagnosis). For example, pancreatic cells, which are identified as malignant tumor cells or normal cells through the cell staining by performing steps (i) and (ii) before the detection of MRS, can be determined more accurately to correspond to pancreatic cancer or be normal by further re-analyzing the expression level (or expression or non-expression) of the MRS protein in the MRS detection step that is subsequently performed, leading to a significant reduction in diagnostic error. Alternatively, pancreatic cells, which are identified as atypical cells through the cell staining, can be clearly determined to correspond to a tumor or not by analyzing the expression level (or expression or non-expression) of MRS in the MRS detection step. As such, a definitive diagnosis with high accuracy can be made at the cellular level, thereby dramatically resolving problems of conventional pathological examinations, for example, the troubles of again performing a biopsy to carry out a re-diagnosis upon the receipt of examination results indicating atypical cells, and the physical burden on a patient attributable to the need for multiple tissue specimens for accurate definitive determination of pancreatic cancer from a biopsy tissue sample, so that a very excellent diagnostic effect can be produced.

The level of MRS detection (level of MRS expression, especially, a level of MRS increase), which is a standard for diagnosis of pancreatic cancer, may be determined by the presence or absence of detection (expression) or by grading the level of detection (expression) according to a measurement method selected by those skilled in the art. For example, a normal range, a pancreatic cancer occurrence range, and the like are classified according to the level of MRS detection (expression) by measuring the expression levels of MRS in samples of multiple normal persons and patients, followed by data storage and analysis, so that an appropriate criterion for diagnosis can be provided.

In addition, the method may be performed in comparison with a negative control sample (especially, a normal control). Therefore, the method may further comprise a step of, after the detection of MRS (the measurement of the expression level of MRS), comparing the level of the MRS protein detected in the pancreatic sample collected from the latent patient with a negative control sample. As used herein, the term "normal control" is meant to encompass all of a pancreatic sample collected from a normal site in the pancreas of a latent patient to be examined (the same individual as the patient to be examined in step (a)) or a pancreatic sample collected from another normal individual (an individual without pancreatic cancer). If the level of MRS protein detected in the pancreatic sample collected from the latent patient is higher than the level in the negative control (especially, a normal control), the latent patient can be determined to be a pancreatic cancer patient.

The information about such a control (e.g., the intensity of detection or expression) may be provided in the form specified in an agent for measuring the expression level of MRS and a kit comprising the same, which are provided in the present disclosure described later, or may be provided subordinately in another form. If the expression level of MRS in a sample to be examined is higher compared with such a control, the sample can be determined to correspond to a pancreatic cancer patient.

As used herein, the term "normal pancreatic cells" or "normal control" refers to non-tumoral (cancerous) pancreatic cells, and the term is meant to encompass all of pancreatic cells in a state of not a tumor (cancer) but a difference disease (e.g., pancreatitis), benign cells, and fully healthy pancreatic cells (disease-free cells) .

In addition, the present disclosure can provide a method for discriminating atypical cells into cancer cells (malignant tumor cells) and normal cells (non-cancer cells) by comprising steps (a) and (b).

As used herein, the term "sensitivity" refers to a proportion of the determination of pancreatic cancer made through a target examination (e.g., an examination of the present disclosure) in a sample or patient having a final clinicopathological diagnosis of pancreatic cancer.

As used herein, the term "specificity" refers to a proportion of the determination of being normal made through a target examination (e.g., an examination of the present disclosure) in a sample or patient having a final clinicopathological diagnosis of being normal.

Therefore, the present disclosure provides a method according to independent claim 1.

It would be obvious to a person skilled in the art that the improvements in sensitivity and specificity lead to an improvement in accuracy. Therefore, the method of the present disclosure can be understood as a method for improving accuracy, and the use of MRS as a marker may lead to an accuracy of 80-100%, preferably 82-98%, and more preferably 85-95%. A specific value for the level includes all of range values each having, as boundary values, two numbers selected from the group consisting of 80%, 81%, 82%, 83%, 84%, 85%, 86%, 87%, 88%, 89%, 90%, 91%, 92%, 93%, 94%, 95%, 96%, 97%, 98%, 99%, and 100%. In one embodiment of the present disclosure, out of the numerical ranges, specifically, 80% and 90% may be selected as boundary values, and thus it would be obvious to a person skilled in the art that all of the values in the range of 80% to 90% are intended in the present disclosure. In still another embodiment, the accuracy may be preferably 80-88%, but is not limited thereto.

Furthermore, the present disclosure provides a method for providing information necessary for diagnosis of pancreatic cancer (a method for diagnosis of pancreatic cancer) by performing a method for discrimination of pancreatic cancer cells in combination with a morphological examination in cytodiagnosis or biopsy for pancreatic cancer, wherein the method for discrimination of pancreatic cancer cells comprises the method of independent claim 1.

The morphological examination is meant to encompass, as a preferable example, an examination conducted by comprising the foregoing steps (i) and (ii), and encompass all of other morphological examinations following such a manner. A description thereof will be made with reference to the above details, and a person skilled in the art could use the above manner through appropriate selection.

The method comprising steps (a) and (b), when performed adjunctively (i.e., as an adjuvant therapy) in addition to a morphological examination, may be performed simultaneously with, separately from, or sequentially with the morphological examination. In addition, the method comprising steps (a) and (b) may be performed before, simultaneously with, or after the morphological examination.

As described above, MRS has an excellent pancreatic tumor (cancer) diagnostic effect as a marker for cytodiagnosis. Therefore, the present disclosure provides a composition for diagnosis of pancreatic cancer, the composition comprising an agent for measuring the expression level of a methionyl-tRNA synthetase (MRS) protein, or a kit for diagnosis of pancreatic cancer.

In addition, the present invention provides use of a composition as defined in independent claim 6.

As used herein, the term "antibody" refers to an immunoglobulin specifically binding to an antigenic site. More specifically, the term indicates a glycoprotein comprising at least two heavy (H) chains and at least two light (L) chains, which are held together by disulfide bonds. Each of the heavy chains is composed of a heavy chain variable region (abbreviated herein as HCVR or VH) and a heavy chain constant region. The heavy chain constant region is composed of three domains, CH1, CH2 and CH3. Each of the light chains is composed of a light chain variable region (abbreviated herein as LCVR or VL) and a light chain constant region. The light chain constant region is composed of one domain, CL. The VH and VL regions can be further subdivided into hypervariable regions (termed complementarity determining regions (CDRs)), with further conserved regions called framework regions (FRs) distributed therebetween. VH and VL each are composed of three CDRs and four FRs, arranged from the amino-terminus to the carboxy-terminus in the following order: FR1, CDR1, FR2, CDR2, FR3, CDR3, and FR4. The variable regions of the heavy and light chains contain binding domains that interact with an antigen. The constant regions of an antibody may mediate the binding of an immunoglobulin to host tissues or factors, including various cells of the immune system (e.g., effector cells) and the first component (C1q) of the classical complement system.

An anti-MRS antibody in the present disclosure is an antibody that specifically binds only to an MRS protein but does not respond to the other proteins including different types of aminoacyl-tRNA synthetases. The antibody specifically binding to the MRS protein in the present disclosure may be preferably an antibody specifically binding to a protein (MRS) comprising an amino acid sequence defined by SEQ ID NO: 1. The anti-MRS antibody may be produced by an ordinary method in the art, for example, the MRS gene is cloned into an expression vector to obtain a protein encoded by the gene and the protein thus obtained is injected into an animal to produce the antibody. The MRS antibody may be produced through the MRS full-length sequence protein. Alternatively, an MRS protein-specific antibody can be produced using an MRS protein fragment comprising an MRS antigenic site. The specific sequence and form of the antibody of the present disclosure are not particularly limited, and include a polyclonal antibody or a monoclonal antibody. In addition, the antibody is not particularly limited to the type of immunoglobulin as provided, and for example, may be selected from the group consisting of IgG, IgA, IgM, IgE, and IgD, and may be preferably an IgG antibody. Furthermore, the antibody of the present disclosure includes special antibodies and recombinant antibodies, such as a humanized antibody and a chimeric antibody, as long as the antibody can specifically bind to an MRS protein. In addition, a part of the whole antibody is also included in the antibody of the present disclosure as long as the part has antigen-antibody binding properties (response), and all types of immunoglobulin antibodies that specifically bind to MRS are included in the antibody of the present disclosure. For example, the antibody of the present disclosure may include not only the full-form antibody having two full-length light chains and two full-length heavy chains but also functional fragments of the antibody molecule, that is, Fab, F(ab'), F(ab')₂, Fv, diabody, scFv, and the like, which have an antigen-binding function.

Fab (fragment antigen-binding) is an antigen-binding fragment of an antibody, and is composed of a heavy chain and a light chain each consisting of one variable domain and one constant domain. F(ab')₂ is a fragment produced by pepsin hydrolysis of an antibody, and F(ab')₂ has a form in which two Fab molecules are linked via disulfide bonds at the heavy-chain hinge region. F(ab') is a monomeric antibody fragment in which a heavy-chain hinge is added to a Fab separated from F(ab')₂ fragment by the reduction of disulfide bonds thereof. Fv (variable fragment) is an antibody fragment composed of only respective variable regions of the heavy and light chains. scFv (single chain variable fragment) is a recombinant antibody fragment in which a heavy chain variable region (VH) and a light chain variable region (VL) are linked to each other via a flexible peptide linker. The term "diabody" refers to a fragment in which VH and VL of scFv, which cannot bind to each other due to the linkage thereof via a very short linker, bind to VL and VH of another scFv in the same form, respectively, to form a dimer. For the purpose of the present disclosure, the fragment of the antibody is not limited to the structure or form thereof as long as the fragment of the antibody retains binding specificity to the human-derived MRS protein.

In the present disclosure, the anti-MRS antibody (including functional fragments thereof) is not particularly limited to the site of MRS, which the antibody interacts with (that is, binds to) as long as the antibody can specifically bind to the MRS protein, but preferably, the antibody may be an antibody or a functional fragment thereof, which specifically binds to an epitope region comprising an amino acid sequence defined by SEQ ID NO: 2 in MRS. More preferably, the antibody of the present disclosure may be an antibody or a fragment thereof, which specifically binds to an epitope containing the 861st to 900th amino acid region in the methionyl-tRNA synthetase (MRS) protein defined by SEQ ID NO: 1.

In an example of the present disclosure, the present inventors verified that for high-sensitivity detection (staining) for MRS in pancreatic cancer cells, an antibody having, as an epitope, a region of an amino acid sequence defined by SEQ ID NO: 2 in MRS was obtained, and that such an antibody could offer high-sensitivity detection ability for MRS.

The antibody specifically binding to an epitope region comprising an amino acid sequence defined by SEQ ID NO: 2 is not particularly limited to the specific sequence thereof as long as the antibody has desired specific binding ability, but preferably, the antibody may comprise:
a light chain variable region (VL) comprising: light chain complementarity-determining region 1 (CDR1) comprising an amino acid sequence defined by SEQ ID NO: 4 or SEQ ID NO:16; a light chain complementarity-determining region 2 (CDR2) comprising an amino acid sequence defined by SEQ ID NO: 6 or SEQ ID NO: 18; and a light chain complementarity-determining region 3 (CDR3) comprising an amino acid sequence defined by SEQ ID NO: 8 or SEQ ID NO: 20, and
a light chain variable region (VH) comprising: heavy chain complementarity-determining region 1 (CDR1) comprising an amino acid sequence defined by SEQ ID NO: 10 or SEQ ID NO: 22; a heavy chain complementarity-determining region 2 (CDR2) comprising an amino acid sequence defined by SEQ ID NO: 12 or SEQ ID NO: 24; and a heavy chain complementarity-determining region 3 (CDR3) comprising an amino acid sequence defined by SEQ ID NO: 14 or SEQ ID NO: 26.

In the antibody (including functional fragments thereof) of the present disclosure, as a preferable example having the CDR configuration, the light chain variable region may comprise an amino acid sequence defined by SEQ ID NO: 28 and the heavy chain variable region may include the amino acid sequence defined by SEQ ID NO: 30.

In the antibody (including functional fragments thereof) of the present disclosure, as another preferable example having the CDR configuration, the light chain variable region may comprise an amino acid sequence defined by SEQ ID NO: 32 and the heavy chain variable region may comprise an amino acid sequence defined by SEQ ID NO: 34.

As the most preferable example, the present disclosure provides an antibody composed of a light chain consisting of the amino acid sequence of SEQ ID NO: 36 and a heavy chain consisting of the amino acid sequence of SEQ ID NO: 37.

As another most preferable example, the present disclosure provides an antibody composed of a light chain consisting of the amino acid sequence of SEQ ID NO: 38 and a heavy chain consisting of the amino acid sequence of SEQ ID NO: 39.

In the present disclosure, detecting agents (typically, antibodies and functional fragments thereof) may be labeled with general detectable moieties for "detection" thereof. For example, the detection agents may be labeled with a radioisotope or a fluorescent label by using the technique described in literature [Current Protocols in Immunology, Volumes 1 and 2, 1991, Coligen et al., Ed. Wiley-Interscience, New York, N. Y., Pubs]. In addition, various enzyme-substrate labels are usable, and examples of the enzymatic label include: luciferase, such as drosophila luciferase and bacterial luciferase (U.S. Patent No. 4,737,456), luciferin, 2,3-dihydrophthalazine dionese, malate dehydrogenase, urase, peroxidase such as horseradish peroxidase (HRPO), alkaline phosphatase, β-galactosidase, glucoamylase, lysozyme, saccharide oxidase (e.g., glucose oxidase, galactose oxidase, and glucose-6-phosphate dehydrogenase), heterocyclic oxidase (e.g., uricase and xanthine oxidase), lactoperoxidase, microperoxidase, and the like. Techniques for conjugating enzymes to antibodies are described in, for example, literature [O'Sullivan et al., 1981, Methods for the Preparation of Enzyme-Antibody Conjugates for use in Enzyme Immunoassay, in Methods in Enzyme. (J. Langone & H. Van Vunakis, eds.), Academic press, N. Y., 73: 147-166]. The labels may be directly or indirectly conjugated to antibodies by using various known techniques. For example, an antibody may be conjugated to biotin, and any labels pertaining to three classes of widespread categories cited above may be conjugated to avidin or vice versa. Biotin may selectively bind to avidin, and therefore, this label may be conjugated to an antibody in such an indirect manner. Alternatively, in order to attain the indirect conjugation of a label to an antibody, the antibody may be conjugated to a small hapten (e.g., digoxin), and one of the different types of labels set forth above may be conjugated to an anti-hapten antibody (e.g., an anti-digoxin antibody). Therefore, the indirect conjugation of labels to antibodies can be attained.

As used herein, the term "aptamer" refers to a substance capable of specifically binding to an analyte to be detected in a sample, wherein the aptamer means a single-stranded nucleic acid having a stable three-dimensional structure *per se* (DNA, RNA, or a modified nucleic acid), and the presence of a target protein in a sample can be specifically identified by an aptamer. An aptamer may be prepared according to a general aptamer preparation method by determining and synthesizing a sequence of an oligonucleotide having selectivity and high binding ability to a target protein to be identified and then modifying the 5'-terminus or 3'-terminus of the oligonucleotide to have -SH, -COOH, -OH, or -NH₂ so as to bind the 5'-terminus or 3'-terminus to a functional group of an aptamer chip, but is not limited thereto.

In order to measure the expression level of the MRS protein, the kit for diagnosis of pancreatic cancer of the present disclosure may comprise not only an antibody or aptamer selectively recognizing an MRS protein but also one or more kinds of other constituent compositions, solutions, or devices suitable for analysis.

In a specific aspect, the kit may be a diagnostic kit comprising known essential elements and subsidiary elements needed for performing western blotting, ELISA, radioimmunoassay, radioimmunodiffusion, Ouchterlony immunodiffusion, rocket immunoelectrophoresis, immunohistostaining assay, immunoprecipitation assay, complement fixation assay, FACS, SPR, or a protein chip assay, but is not limited thereto.

For example, the kit may comprise an antibody specific to an MRS protein. The antibody may be a monoclonal, polyclonal, or recombinant antibody, which has high specificity and affinity to a target marker protein and has little cross-reactivity to other proteins. In addition, the kit may comprise an antibody specific to any control protein. The antibody provided in the kit may itself be labeled with a detectable moiety, which is as described above. Additionally, the kit may further comprise a separate reagent capable of detecting a bound antibody, for example, a labeled secondary antibody, a chromophore, an enzyme (e.g., in the form of being conjugated with the antibody) and a substrate thereof, or other materials capable of binding to the antibody. In addition, the kit of the present disclosure may comprise a wash or an eluent, which can remove excess chromogenic substrates, unbound proteins, and the like but retain only a protein marker bound to an antibody.

The present inventors found that in the diagnosis of pancreatic cancer, MRS was not expressed in most of normal cells, but a small number of normal cells often showed a false positive by MRS expression, and such a false-positive determination resulted from a high expression of MRS in normal acinar cells. As a result of testing various marker combinations in order to exclude such false-positive reactions and find a method with higher accuracy (including sensitivity and specificity), a dual staining method for MRS and CK19 of the present disclosure was developed. The present inventors first established that when CK19, besides MRS, is specifically used as an additional double marker in the diagnosis of pancreatic cancer, the diagnostic accuracy of pancreatic cancer in cytodiagnosis is substantially (almost) 100%. Therefore, a sample classified as atypical cells in conventional cytodiagnosis can be discriminated into pancreatic cancer and non-pancreatic cancer (normal).

Accordingly, the present disclosure provides a method for detecting methionyl-tRNA synthetase (MRS) and cytokeratin 19 (CK19) proteins in a pancreatic sample collected from a latent patient, in order to provide information necessary for the diagnosis of pancreatic cancer. That is, the present disclosure provides a method for diagnosis of pancreatic cancer by measuring the expression levels of methionyl-tRNA synthetase (MRS) and cytokeratin 19 (CK19) proteins in a sample of a subject.

Specifically, the method may comprise:
**(a) measuring the expression levels of MRS and CK19 proteins in a pancreatic sample collected from a latent patient; and**
**(b) determining the latent patent to be a pancreatic cancer patient when the CK19 protein is expressed and the expression level of the MRS protein is increased in step (a).**

As used herein, "CK19" is meant to indicate cytokeratin 19, and CK19 is an intermediate filament protein belonging to type I keratin and is mainly expressed in epithelial cells and is responsible for structural integrity of the epithelial cells. The CK19 protein of the present disclosure is particularly limited to the specific sequence and a biological origin thereof as long as the CK protein includes the CK19 amino acid sequence known in the art. For example, CK19 is encoded by the KRT19 gene in humans, and the sequence information of CK19 is known by a Genbank accession number, such as NM_002276.4 (mRNA) or NP_002267.2 (protein). Preferably, the CK19 of the present disclosure may comprise an amino acid sequence of the human CK19 protein defined by SEQ ID NO: 3. More preferably, the CK19 protein of the present disclosure may consist of the amino acid sequence defined by SEQ ID NO: 3.

In the present disclosure, the detection of the MRS and CK19 proteins is not particularly limited to a method therefor as long as the detection is carried out by a protein expression level measurement method known in the art. For example, the MRS and CK19 proteins can be detected or measured using antibodies specifically binding to the proteins, respectively. Specifically, the detection of the proteins in the present disclosure may be carried out by, for example, any one selected from the group consisting of, but is not limited to, western blotting, enzyme linked immunosorbent assay (ELISA), radioimmunoassay, radioimmunodiffusion, Ouchterlony immunodiffusion, rocket immunoelectrophoresis, immunohistostaining (including immunohistochemical staining, immunocytochemical staining, and immunofluorescence staining), immunoprecipitation assay, complement fixation assay, fluorescence activated cell sorter (FACS) assay, surface plasmon resonance (SPR), or protein chip assay.

Specifically, the method for detecting MRS and CK19 proteins in a pancreatic sample collected from a latent patient in order to provide information necessary for the diagnosis of pancreatic cancer may be performed by measuring the presence or absence of the expression of MRS and CK19 proteins or the expression levels of MRS and CK19 proteins in a pancreatic sample collected from a latent patient and determining the latent patient to be a pancreatic cancer patient when the CK19 protein is expressed and the MRS protein is (highly) expressed in the pancreatic sample. The method has an advantage in that a direct determination of pancreatic cancer (especially, pancreatic ductal cancer or pancreatic ductal adenocarcinoma) with very excellent accuracy can be attained through the detection of (high) expression of the MRS protein together with the CK19 protein even without a particular comparison procedure. This is well described in the examples of the invention.

In addition, the determination can be performed through the comparison with a negative control sample (especially, a normal control sample), and such a comparison of the intensity of detection or expression is understood with reference to the above description. Specifically, cells showing an expression of CK19 protein and an increase in expression of MRS protein compared with a negative control can be determined to be pancreatic cancer cells.

Therefore, according to an aspect, the present disclosure provides

A method for diagnosis of pancreatic cancer, the method comprising:
(a) measuring the expression levels of MRS and CK19 proteins in a pancreatic sample collected from a latent patient; and
(b) comparing the expression level of the methionyl-tRNA synthetase protein measured in step (a) with that of a negative control, and determining the latent patient to be a pancreatic cancer patient when the expression level of the methionyl-tRNA synthetase protein is increased compared with that of the negative control and the CK19 protein is expressed.

In particular, it was verified that MRS and CK19 were highly expressed and detected even in pancreatic cells of a patient wherein the pancreatic cells were identified as atypical cells by conventional cytopathological diagnosis (based on H&E staining, pap staining, or the like) and thus undiagnosable but the patient was finally diagnosed with pancreatic cancer as a result of follow-up observation of the patient afterward. Considering that it is very difficult to discriminate whether atypical cells correspond to a tumor or other benign disease by conventional pathological cytology that is generally used for cancer diagnosis, the determination of whether such atypical cells correspond to a tumor is clinically very important, and it is very meaningful that atypical cells can be determined to be tumor cells when a (high) expression of MRS is observed in the atypical cells. In particular, the combinative use of MRS with CK19 notably reduces the error of the false-positive determination due to acinar cells, thereby significantly increasing the accuracy in diagnosis of pancreatic cancer.

Furthermore, considering that compared with cytodiagnosis, a biopsy requiring relatively large amounts of biopsy material increases the physical burden on patients in terms of sample acquisition, and in some cases, surgery is impossible and the collection of large amounts of tissue is impossible depending on the progression of cancer, the method of the present disclosure, which provides an accurate diagnosis even at the cellular level, has even greater advantages.

When the diagnosis of pancreatic cancer employs a manner in which both MRS and CK19 are used as markers and increases thereof are detected, the sensitivity, specificity, positive predictive value and/or negative predictive value is substantially (almost) 100% in the examinations at the tissue and cellular level.

Specifically, at least one selected from the group consisting of the sensitivity, specificity, positive predictive value, and negative predictive value shows a level of 80% or higher (80-100%, preferably 85-99%, and more preferably 90-98%) . A specific value for the level includes all of range values each having, as boundary values, two numbers selected from the group consisting of 80%, 81%, 82%, 83%, 84%, 85%, 86%, 87%, 88%, 89%, 90%, 91%, 92%, 93%, 94%, 95%, 96%, 97%, 98%, 99%, and 100%. In one embodiment of the present disclosure, out of the numerical ranges, specifically, 80% and 95% may be selected as boundary values, and thus it would be obvious to a person skilled in the art that all of the values in the range of 80% to 95% are intended in the present disclosure. In another embodiment, the sensitivity, specificity, positive predictive value and/or negative predictive value shows a level of 85% or higher (85-100%, preferably 87-99%, and more preferably 90-98%), but is not limited thereto.

Therefore, the present disclosure provides a method for improving sensitivity and specificity in a cytodiagnosis or biopsy of pancreatic cancer, the method comprising:
(a) measuring the expression levels of methionyl-tRNA synthetase and cytokeratin 19 (CK19) proteins in a pancreatic sample collected from a latent patient; and
(b) determining the latent patient to be a pancreatic cancer patient when the expression level of the cytokeratin 19 (CK19) protein is detected (expressed) and the expression level of the methionyl-tRNA synthetase protein is increased in step (a).

It would be obvious to a person skilled in the art that the improvements in sensitivity, specificity, positive predictive value and/or negative predictive value lead to an improvement in accuracy. Therefore, the method of the present disclosure can be understood as a method for improving accuracy, and the accuracy may show a level of preferably 90-100%, and more preferably 90-99%. A specific value for the level includes all of range values each having, as boundary values, two numbers selected from the group consisting of 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5%, and 100%. In one embodiment of the present disclosure, out of the numerical ranges, specifically, 92.5% and 99.5% may be selected as boundary values, and thus it would be obvious to a person skilled in the art that all of the values in the range of 93.5% to 99.5% are intended in the present disclosure. In still another embodiment, the accuracy may show a level of more preferably 93-98%, and most preferably 95-98%, but is not limited thereto.

Furthermore, the present disclosure provides a method for providing information necessary for diagnosis of pancreatic cancer (a method for diagnosis of pancreatic cancer) by performing a method for discrimination of pancreatic cancer cells in combination with a morphological examination in cytodiagnosis or biopsy for pancreatic cancer, wherein the method for discrimination of pancreatic cancer cells comprises:
(a) measuring the expression levels of methionyl-tRNA synthetase and cytokerain 19 (CK19) proteins in a pancreatic sample collected from a latent patient; and
(b) determining the latent patient to be a pancreatic cancer patient when the cytokerain 19 (CK19) protein is detected (expressed) and the expression level of the methionyl-tRNA synthetase protein is increased in step (a).

The morphological examination is meant to encompass, as a preferable example, an examination conducted by comprising the foregoing steps (i) and (ii), and to encompass all of other morphological examinations following such a manner. A description thereof will be made with reference to the above details, and a person skilled in the art could use the above manner through appropriate selection.

A specific description of steps (a) and (b) is as described above, and the steps may be performed simultaneously with, separately from, or sequentially with the morphological examination when such steps are performed adjunctively (i.e., as an adjuvant therapy). In addition, the determination method comprising steps (a) and (b) may be performed before, simultaneously with, or after the morphological examination.

In the present disclosure, the agent for measuring the expression level of the CK19 protein is not particularly limited to the kind thereof as long as the agent is known to be usable in the measurement of the expression level of a protein in the art. Preferably, the agent may be an antibody or aptamer specifically biding to the CK19 protein, and a specific description thereof follows the descriptions of the above anti-MRS antibody and aptamer.

### Advantageous Effects

The methionyl-tRNA synthetase (MRS) as a pancreatic cancer marker shows a much higher diagnostic accuracy than a conventional pancreatic cancer marker, such as CEA, so that a clear determination of pancreatic cancer or non-pancreatic cancer can be made by analyzing the expression or non-expression of methionyl-tRNA synthetase (MRS) in pancreatic cells, and an equivalent effect is achieved in even the cells identified as atypical cells by ordinary staining, and thus MRS can be very helpfully used in the diagnosis of pancreatic cancer.

### Brief Description of the Drawings

FIG. 1 shows western blot results comparing the MRS-binding strength and specificity of anti-MRS antibodies (1E8 and 8A12) of the present disclosure with a known commercially accessible MRS antibody (Ab50793) by using cell lysates of H460 cells treated with si-MRS.
FIG. 2 shows western blot results comparing the MRS-binding strength and specificity of anti-MRS antibodies (1E8 and 8A12) of the present disclosure with a known commercially accessible MRS antibody (Ab137105) by using the cell lysates of PANC-1 cell line (pancreatic cancer cell line) and SCK cell line (non-pancreatic cancer cell line).
FIG. 3 shows a graph depicting the results of ELISA conducted to investigate the cross-reactivity of the 1E8 antibody with other aminoacyl-tRNA synthetase (ARS) and AIMP proteins.
FIG. 4 shows a graph depicting the results of ELISA conducted to investigate the cross-reactivity of the 8A12 antibody with other aminoacyl-tRNA synthetase (ARS) and AIMP proteins.
FIG. 5 shows the results of surface plasmon resonance (SPR) tests conducted to investigate antibody affinity of the 1E8 antibody to MRS+AIMP3 protein.
FIG. 6 shows the surface plasmon resonance (SPR) test results verifying that the 1E8 antibody had no response to AIMP3.
FIG. 7 shows the results of surface plasmon resonance (SPR) tests conducted to investigate antibody affinity of the 8A12 antibody to MRS+AIMP3 protein.
FIG. 8 shows the surface plasmon resonance (SPR) test results verifying that the 8A12 antibody had no response to AIMP3.
FIG. 9 compares the immunofluorescence staining results using the anti-MRS antibodies (1E8 and 8A12) of the present disclosure and the commercially accessible MRS antibody (Ab137105) on the PANC-1 cell line (pancreatic cancer cell line) and the SCK cell line (non-pancreatic cancer line).
FIG. 10 shows the results of immunofluorescence staining of the 1E8 and 8A12 antibodies of the present disclosure conducted by manufacture Thinprep slides similar to clinical conditions of the PANC-1 cell line through the Thinprep device (Hologic. Inc) used in patient sample processing in clinical sites.
FIG. 11 shows the H&E staining results, observation results of MRS expression or non-expression, and observation results of CEA expression or non-expression in the pancreatic cells isolated from normal patients (each number represents a patient code number).
FIG. 12 shows the H&E staining results, observation results of MRS expression or non-expression, and observation results of CEA expression or non-expression in the pancreatic cells isolated from pancreatic cancer patients (each number represents a patient code number).
FIG. 13 shows the observation results of MRS expression or non-expression and the observation results of CEA expression or non-expression in the pancreatic cytodiagnosis samples of patients that was identified as atypical cells as a result of H&E staining and afterward was definitively diagnosed with pancreatic cancer (each number represents a patient code number).

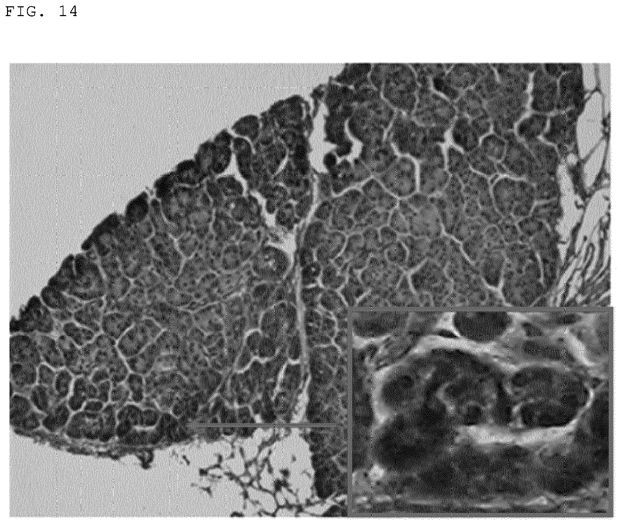

FIG. 14 shows the results of observing the expression level of MRS in normal acinar cells in the normal pancreatic tissue.
FIG. 15 shows the results of double detection of MRS and CK19 proteins in pancreatic cancer cells (PANC-1 cell line), depicting that the staining intensity was shown for each marker.
FIG. 16 shows the results of double detection of MRS and CK19 proteins in non-pancreatic cancer cells (CT26 cell line), depicting that each marker was not detected.
FIG. 17 shows staining images by conventional pathologic cytology (pap staining) and the results of double staining of the present disclosure, in the cell sample containing a plurality of pancreatic acinar cells.
FIG. 18 shows the results of the double staining of the present disclosure in the pancreatic cell sample of a patient classified as atypical cells by conventional pathologic cytology (pap staining) and thus undiagnosable but finally diagnosed with pancreatic cancer.
FIG. 19 shows the results of the double staining of the present disclosure in the pancreatic cell sample of a patient undiagnosed due to being suspicious of pancreatic cancer (malignancy) by conventional pathologic cytology (pap staining) but finally diagnosed with pancreatic cancer.
FIG. 20 shows the results of the double staining of the present disclosure in the pancreatic cell sample of a patient diagnosed with pancreatic cancer by conventional pathologic cytology and finally diagnosed with pancreatic cancer.
FIG. 21 shows the results of MRS detection of the present disclosure and additional CK19 detection in the tissue (in which pancreatic cancer cells co-exist with normal cells) determined to be a pancreatic cancer tissue by conventional pathologic histology (H&E staining).
FIG. 22 shows the results of MRS detection of the present disclosure and additional CK19 detection in the tissue determined to be normal pancreatic tissue by conventional pathologic histology (H&E staining) (arrows indicating pancreatic duct regions stained with CK19).

### Mode for Carrying Out the Invention

Hereinafter, the present disclosure will be described in detail.

However, the following examples are merely for illustrating the present disclosure, and are not intended to limit the scope of the present disclosure.

### Example 1: Construction of useful antibody for present inventive method for examination of pancreatic cancer (Obtaining antibody having high specificity to MRS)

It has been known that in vivo, methionyl-tRNA synthetase (MRS) is present in a state of binding with aminoacyl-tRNA synthetase complex-interacting multifunctional protein 3 (AIMP3) and such binding is broken by UV irradiation or the like. Therefore, for substantially accurate detection of MRS, only MRS needs to be specifically detected even in situations where MRS binds with AIMP3. However, current AIMP types and ARS types have many similarities in terms of protein structures, and thus commercial antibodies have a problem of showing cross-reactivity with other AIMP and ARS types. For diagnostic accuracy in the pancreatic cancer examination method of the present disclosure, the present inventors produced high-sensitivity MRS antibody having no cross-activity with the other proteins as below.

### 1-1. MRS-AIMP3 protein production

MRS-AIMP3 co-purified protein was expressed and purified from *E. coli,* and specific experiment methods are as follows. BL21DE3 strain was transformed so as to express MRS (SEQ ID: 1) and AIMP3 (SEQ ID NO: 40, NCBI ref.NM_004280.4), and cultured in LB medium, and then single colony was cultured to reach an OD600 value of 0.6-0.8 in 5 ml of LB liquid medium containing ampicillin. After 1 mM IPTG was added, the cells were incubated at 37°C for 3 h, and then only the cells were obtained by centrifugation for 10 minutes. SDS-PAGE was performed with the cell solution to check for the expression of the proteins using Coomassie staining. Thereafter, the cell solution having IPTG-induced overexpression was collected and centrifuged to obtain cells. The cells were loosened with 1 ml of DPBS, followed by cell lysis using an ultrasonicator, and then the lysed cells were centrifuged to separate MRS-AIMP3 co-purified protein.

### 1-2. Mouse immunization through injection of MRS-AIMP3 protein

To obtain immunized mice necessary for the preparation of hybridoma cells, the MRS-AIMP3 co-purified protein obtained in Example 1-1 was primarily injected into the abdominal cavity of four 8- to 10-week old mice. The 10-week old BALB/c mice weighing 25-30 g were purchased from Orient Bio Co. (Sungnam, KyungKi-Do, Republic of Korea). The animals were sufficiently acclimated under predetermined conditions (temperature: 20±2°C, humidity: 40-60%, light cycle: 12-h light/dark cycle), and then used in the present study. Animal experiments were conducted according to the guidelines of the Institutional Animal Care and Use Committee of Seoul National University. Two weeks later, the same dose of MRS-AIMP3 co-purified protein was secondarily injected into the abdominal cavity of the mice to enhance the immunity of the mice after the primary immunization. One week later, the MRS-AIMP3 co-purified protein was booster-injected into the tail vein of the mice three days before cell fusion. After the immunized mice were anesthetized with ether, blood was drawn from the heart using a heparinized syringe. Thereafter, the blood was allowed to stand overnight at 4°C, and then centrifuged to separate serum. The separated serum was appropriately divided and stored at -80°C.

### 1-3. Preparation of hybridoma cells

First, myeloma cells were prepared for cell fusion. The myeloma cells were cultured to a cell density of 2.5 to 5 × 10⁴ cell/ml. The myeloma cells were prepared by a 1/3 dilution 24 hours before cell fusion. The mice immunized in Example 1-2 were anesthetized with ether, and then spleens were harvested, followed by isolation of B cells. The spleens were washed with SF-DMEM2 (DMEM + 2×AA), followed by cell elution. The cell suspension was collected, placed in a tube, and allowed to stand to settle heavy masses. The supernatant was transferred to a new tube, and then centrifuged at 1500 rpm for 5 m. The centrifuged splenocytes were collected by removing the supernatant, and the tube was tapped and then filled with SF-DMEM2. The B cells and the myeloma cells were separately centrifuged and washed, and then washing was repeated once more. The supernatant of the washed myeloma cells was removed, and the tube was tapped and then filled with SF-DMEM2. In addition, the supernatant of the washed B cells was removed, and the tube was tapped, and treated with 1 ml of lysis buffer (LB) to lyse red blood cells (RBCs), and then filled with SF-DMEM2. Then, the B cells and the myeloma cells were separately centrifuged, and the supernatants of the centrifuged splenocytes and myeloma cells were removed, and then the tubes were tapped and filled with 10 ml of SF-DMEM2. The B cells and myeloma cells were diluted 100-fold in e-tubes, respectively, and counted to determine concentrations thereof [B cell concentrations (1×10⁸, 8×10⁷, 5×10⁷), myeloma cell concentrations (1×10⁷, 8×10⁶, 5×10⁶)]. The B cells and the myeloma cells were determined at a ratio of 10:1. The B cells and the myeloma cells with the determined concentrations were placed together in a tube and centrifuged. The supernatant of the centrifuged cells was removed, and then the tube was put upside down on an alcohol pad and semi-dried for 30 s to 1 min, and tapped. PEG (2 ml) was slowly added for 1 min to the tube while pipetting, and the tube was shaken with the addition of SF-DMEM2, followed by centrifugation. After the centrifugation, the supernatant was removed and, without tapping, HT medium [1 ml of HT50×(HT(sigma) 1 vial + SF-DMEM1 10 ml), 10 ml of FBS, 30 ml of SF-DMEM1 (DMEM + 1×AA)] was added dropwise with gradually increasing speed, to reach 50 ml. This suspension was again incubated in a 5% CO₂ incubator at 37°C for 3 h.

### 1-4. Selection of hybridoma cells producing MRS-specific monoclonal antibodies

To select cells specifically recognizing MRS but not AIMP3 in the fusion cell groups prepared in Example 1-3 above and investigate the production or non-production of an antibody, the following test was conducted.

First, the medium was exchanged 8-9 days after cell fusion, and incubated in cDMEM2 until the cells were well grown in 96 wells and 24 wells. After medium exchange, the supernatants in wells in which the color has changed were collected and filled with cDMEM2 on day 5-7, and then an ELISA test was performed for the binding of an antibody produced from each fusion cell with MRS and AIMP3. After the ELISA test, wells were selected, and the cells in the selected well were transferred in 24 wells, followed by incubation. After the incubation in 24 wells, an ELISA test was again performed. Specifically, the concentration of fusion cells in 24 wells was checked, and the fusion cells were diluted in 15 ml of the culture so as to reach 0.5 cells/well in the 96-well plate. The fusion cell dilution was dispensed at 150 µl per well. The wells containing one cell were checked by microscopy. The supernatant in the wells containing cells that grew to some extent was collected, and primary screening was conducted by investigating the binding of the antibody, produced from respective fusion cells, with MRS and AIMP3 through ELISA and western blotting. The fusion cells selected on the basis of the primary screening were transferred and incubated in 24 wells, followed by centrifugation, and thereafter, the supernatant was collected, and secondary screening was conducted by ELISA and western blotting. The absorbance (OD value) of the fusion cells grown in the 24 wells was checked by ELISA to select only fusion cells having an absorbance exceeding 1.0. The selected cells were transferred and incubated in 25T/C culture flask and centrifuged, and then the supernatant was collected, and tertiary screening was conducted by ELISA and western blotting. The fusion cells selected on the basis of the tertiary screening were again transferred and incubated in 75T/C culture flask, and then the absorbance was checked by ELISA to select cells well recognizing MRS but not AIMP3, thereby finally securing "1E8" and "8A12" clones.

### 1-5. Culture of hybridoma cells producing MRS-specific monoclonal antibodies and purification of antibodies

Monoclonal antibodies to MRS can be obtained from the final fusion cells (hybridoma cells "1E8" and "8A12") selected in Example 1-4 by the following two methods.
1) Female mice aged 7-8 weeks were injected with 500 µl of pristane through the abdominal cavity. The fusion cells cultured in the 75T/C culture flask were collected and centrifuged, followed by supernatant removal, and then the cells were placed in a phosphate buffer and pipetted. After 7-10 days of pristane administration, the fusion cells selected in Example 1-4 were injected in an amount of 8×10⁵ to 4×10⁷ cells into the abdominal cavity of the mice. When the abdominal cavity of the mice was full of ascites 12 weeks later, the ascites were extracted using an 18G-syringe needle. The ascites were kept at 4°C overnight and then centrifuged the next day, thereby removing a mass material containing a yellow layer of fat and separating only the supernatant. The separated supernatant was dispensed and stored at -20°C.

For the purification of antibodies from the ascites, a column was packed with an appropriate amount of protein A, which has been stored in a stock solution (20% ethanol), and 20% ethanol was allowed to flow through the column, and then the column was washed with a 5-bed volume of a binding buffer (20 mM sodium phosphate, pH 7.0). The ascites were diluted with an appropriate amount of a phosphate buffer, and was then loaded on the protein A column. After binding was conducted using a 3-bed volume of a binding buffer (20 mM sodium phosphate, pH 7.0), 5 ml of fractions were eluted with a 3-bed volume of an elution buffer (0.1 M glycine buffer, pH 3.0-2.5). Each fraction was neutralized with 35 µl of a neutralization buffer (1M Tris-HCl, pH 9.0). The fractions were checked for purity through SDS-PAGE, and desalted with Ammersharm GE column.

2) The hybridoma cells obtained in Example 1-4 were acclimated in serum-free medium (Thermo) supplemented with GlutaMAX (Gibco) (final 5 mM) and 1x cholesterol lipid concentrate (Gibco) (8A12 antibody-producing hybridoma cells are also named 34-8F2). Thereafter, the cells were cultured in a maximum culture volume of 860 ml using Cellstack-5 (Corning, Corning, NY). GlutaMAX (Gibco) (final 5 mM) and 1x cholesterol lipid concentrate (Gibco) were added to the serum-less medium (Thermo), and the cells were inoculated at an initial cell concentration of 1.4-2.0 × 10⁵ cells/ml. After 4-5 days of the inoculation, the cells were removed by centrifugation at 2000 rpm for 10 m and the culture supernatant was recovered. The pH of the supernatant was checked, and then the pH was adjusted to 7.6 using the prepared 20X binding solution (1M potassium phosphate dibasic, pH 9.0). Thereafter, the supernatant was filtered using a 0.22-µm filter to obtain a neutralized antibody culture solution.

The obtained antibody culture solution was purified through a protein A column. After 10 column volumes of distilled water were allowed to flow through the protein A column, an equal amount of 1× binding solution (50 mM potassium phosphate dibasic, pH 9.0) was allowed to flow therethrough. Thereafter, the obtained antibody culture solution was allowed to flow therethrough to bind antibodies to protein A, followed by washing with 1× binding solution (50 mM potassium phosphate dibasic, pH 9.0). To elute the antibodies bound to protein A, 2 column volumes of an elution solution (0.2 M citric acid, pH 3.0) were allowed to flow therethrough, thereby obtaining an eluate. After the eluate was neutralized with 1 M Tris, the concentration of antibodies was determined by measurement of the absorbance at 280 nm.

Thereafter, the GE PD-10 column was equilibrated with 25 ml of physiological saline and then centrifuged (1,000 g, 2 min). Thereafter, 2.5 ml of the antibody eluate obtained from the protein A column was added to the GE PD-10 column, followed by centrifugation (1000 g, 2 min), thereby collecting an antibody solution exchanged with physiological saline. The antibody concentration was determined by measurement of the absorbance at 280 nm, and then the antibody solution was dispensed and stored at -80°C.

### 1-6. Antibody sequencing and cloning

The 1E8 and 8A12 clone-expression antibodies were cloned and sequenced by YBIO Inc. and Abclon Inc. (Korea), respectively. Briefly, RNA was first extracted from 1E8 or 8A12 hybridoma cells to synthesize cDNA. Then, PCR was performed using primers specific to VL, CL, VH, and CH1. PCR products with expected sizes were purified on agarose gel, the sequences thereof were identified through sequencing, and CDR regions were identified through Kabat numbering. The sequencing results of the antigen-binding region of 1E8 antibody are shown in Table 1, and the sequencing results of the antigen-binding region of 8A12 antibody are shown in Table 2. The Fab molecules were synthesized from the identified sequences, and were verified to show high binding ability to MRS through ELISA.

It was also verified that the above identified sequences are consistent with the protein sequencing results (mass spectrometry results) of the antibodies, which were obtained through ascites purification after the hybridoma cells were injected into the abdominal cavity of the mice in Example 1-5.

The obtained 1E8 Fab or 8A12 Fab sequences were cloned into the mouse IgG heavy chain sequence vector (pFUSE-mIgG2a-Fc, InvivoGen) and mouse light chain (pFUSE2-CLIg-mK, InvivoGen) sequence vector. Then, the vectors were co-transformed into freestyle 293F cells using PEI (Polysciences, 23966-2), so that antibody heavy and light chains were co-expressed together in the cells. The transformed 293F cells were cultured under conditions of 37°C and 8% CO₂ for 7 days. Then, the cells were obtained and centrifuged, and the supernatant was obtained. The pH of the supernatant was checked, and then the pH of the supernatant was adjusted to 7.6 using the prepared 20X binding solution (1M potassium phosphate dibasic, pH 9.0). Thereafter, the supernatant was filtered using a 0.22-um filter to obtain a neutralized antibody culture solution. An antibody was obtained from the antibody culture solution by the method described in 2) of Example 1-5. It was confirmed that the whole 1E8 IgG antibody thus obtained is composed of a light chain comprising an amino acid sequence of SEQ ID NO: 36 and a heavy chain comprising an amino acid sequence of SEQ ID NO: 37. It was also confirmed that the whole 8A12 IgG antibody thus obtained is composed of a light chain comprising an amino acid sequence of SEQ ID NO: 38 and a heavy chain comprising an amino acid sequence of SEQ ID NO: 39.

**[Table 1]**

| | | Amino acid sequence | DNA sequence |
|---|---|---|---|
| VH | FR1 | | |
| | CDR-H1 | SDYAWN (SEQ ID NO: 10) | Agtgattatgcctggaac (SEQ ID NO: 11) |
| | FR2 | WIRQFPGNKLEWMG (SEQ ID NO: 43) | |
| | CDR-H2 | YISYSGRTSYKSSLKS (SEQ ID NO: 12) | |
| | FR3 | | |
| | CDR-H3 | DYGNFVGYFDV (SEQ ID NO: 14) | |
| | FR4 | WGAGTTVTVSS (SEQ ID NO: 47) | |
| VL | FR1 | | |
| | CDR-L1 | KSSQSLLYSSNQKNYLA (SEQ ID NO: 4) | |
| | FR2 | WYQQKPGQSPKLLIY (SEQ ID NO: 51) | |
| | CDR-L2 | WASTRES (SEQ ID NO: 6) | Tgggcatccactagggaatct (SEQ ID NO: 7) |
| | FR3 | | |
| | | | |
| | CDR-L3 | QQYYSYPT (SEQ ID NO: 8) | |
| | FR4 | FGGGTKLEIK (SEQ ID NO: 55) | |

**[Table 2]**

| | | Amino acid sequence | DNA sequence |
|---|---|---|---|
| VH | FR1 | | |
| | CDR-H1 | SEYAWT (SEQ ID NO: 22) | Agtgagtatgcctggacc (SEQ ID NO: 23) |
| | FR2 | WIRQFPGNKLEWMG (SEQ ID NO: 59) | |
| | CDR-H2 | YINYNGNTNLNPSLKS (SEQ ID NO: 24) | |
| | FR3 | | |
| | CDR-H3 | SLWPRGWFAY (SEQ ID NO: 26) | |
| | FR4 | WGQGTLVTVSA (SEQ ID NO: 63) | |
| VL | FR1 | | |
| | CDR-L1 | KASQDINSYLS (SEQ ID NO: 16) | |
| | FR2 | WFQQKPGKSPKTLMY (SEQ ID NO: 67) | |
| | CDR-L2 | RANRLVD (SEQ ID NO: 18) | Cgtgcaaacagattggtagat (SEQ ID NO: 19) |
| | FR3 | | |
| | CDR-L3 | LQYDEFPRT (SEQ ID NO: 20) | |
| | FR4 | FGGGTKLEIK (SEQ ID NO: 71) | |

### 1-7. Verification on comparison of binding specificity of antibody to MRS - western blotting

To investigate the MRS binding ability of the 1E8 and 8A12 antibodies obtained in the above example, the following western blot test was conducted. H460 cells were incubated in DMEM (HyClone, GE Life Sciences) containing 10% fetal bovine serum (FBS, HyClone, GE Life Sciences) and 1% penicillin (HyClone, GE Life Sciences). All the cells were incubated under conditions of 5% CO₂ and 37°C. The incubated H460 cells were treated with si-MRS for 72 h. Then, the H460 cells were obtained and lysed, and then the H460 cell lysate was subjected to western blotting. The test was repeated twice. The 1E8 or 8A12 antibody as a primary antibody was diluted to 1:5000 (0.2 µg/ml) before use and, for comparison of binding ability, a currently commercially available MRS antibody (Abcam, Ab50793) was used by the same method, and tubulin was used as a control.

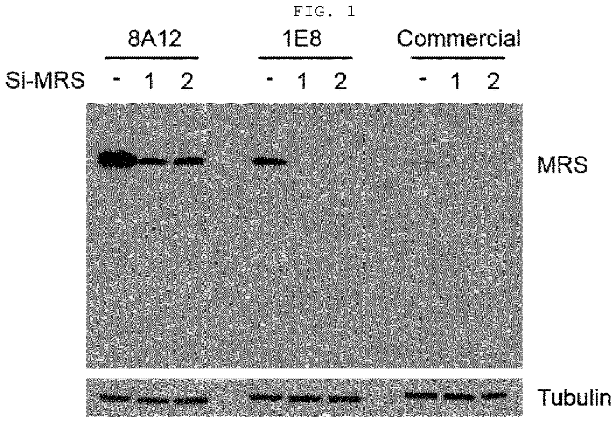

As a result of the test, as shown in FIG. 1, the conventional commercially available MRS antibody never detected MRS in the si-MRS treatment group and showed significantly low detection ability (binding ability with MRS) compared with the 8A12 and 1E8 antibodies of the present disclosure even in the si-MRS non-treatment group. On the contrary, the 8A12 and 1E8 antibodies of the present disclosure showed significantly excellent MRS-specific binding ability and sensitivity compared with the conventional commercially available MRS, and especially the 8A12 antibody showed very excellent binding ability and sensitivity.

In addition, the MRS-binding ability of the 8A12 and 1E8 antibodies was further investigated using the PANC-1 pancreatic cancer cell line and SCK cell line (non-pancreatic cancer cell line). A commercially available MRS antibody (Abcam, Ab137105) was used as a control (antibodies were diluted to 1:1000 before use, 0.137µg/ml), and western blotting was carried out in the same manner as described above except for the si-MRS treatment procedure.

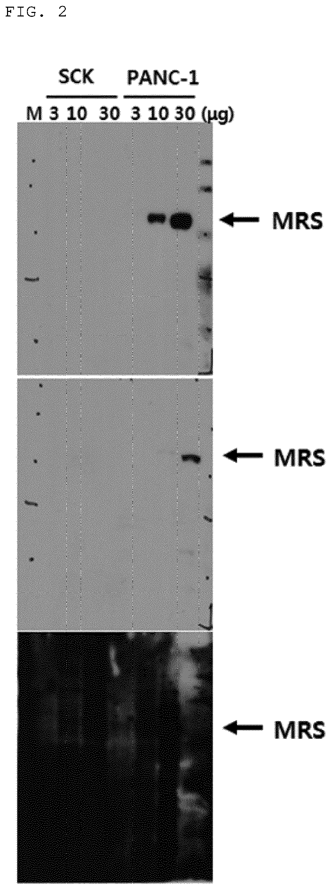

As a result of the test, as shown in FIG. 2, the 8A12 and 1E8 antibodies of the present disclosure made an MRS-specific detection, but in the same conditions, the conventional commercially available antibody Ab137105 showed many non-specific bands, indicating very low selective detectability. Under the present test conditions, MRS was little detected in the SCK cell line (non-pancreatic cancer cell line).

### 1-8. Verification on cross-reactivity with other proteins - ELISA

To investigate whether the 8A12 antibody obtained in the above example had cross-reactivity with other aminoacyl-tRNA synthetase (ARS) proteins, the following test was conducted.

On 96-well plates (Corning 3690 flat bottom, 96-well half-area plates), MRS proteins (His-MRS and MRS full) and other ARS proteins (DX2 tag free, 34S-DX2, 34S-AIMP2, His-CRS, His-AIMP1, His-GRS, His-WRS, and His-KRS) were each coated at a concentration of 1 µg/ml. The 1E8 or 8A12 antibody was added at a concentration of 500 ng/ml to the 96-well plates coated with the respective ARS proteins, followed by incubation for 1 hour. Thereafter, HRP-conjugated anti-mouse IgG secondary antibody was added, followed by incubation for 1 h, and then the absorbance at 450 nm was measured by ELISA. As a substrate, 3,3',5,5'-tetramethylbenzidine (TMB) was used.

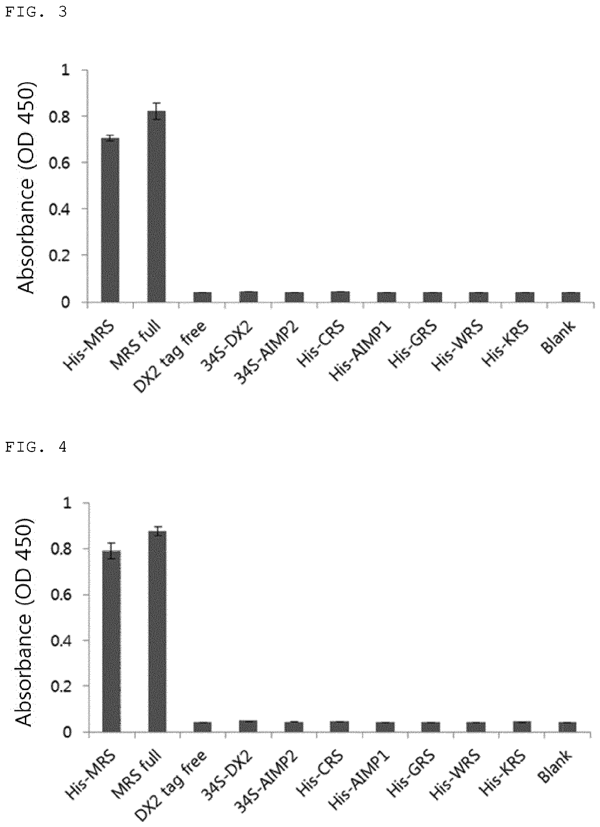

As a result, as shown in FIG. 3, the 1E8 antibody bound to and reacted with only MRS, but did not react with other ARS and AIMP proteins. As a result, as shown in FIG. 4, the 8A12 antibody bound to and reacted with only MRS, but did not react with other ARS and AIMP proteins. The results verified that the 1E8 and 8A12 antibodies had no cross-activity with other ARS and AIMP proteins, and specifically detected only MRS.

### 1-9. Verification on antibody affinity using surface plasmon resonance

To investigate MRS-specific affinity of the 1E8 and 8A12 antibodies, a surface plasmon resonance (SPR) test was conducted using MRS-AIMP3 co-purified protein (hereinafter, MRS+AIMP3 protein) and AIMP3 protein. MRS+AIMP3 or AIMP3 protein was coated on a CM5 chip, and different concentrations of the 1E8 or 8A12 antibody were allowed to flow down, thereby measuring the degree of binding response with the protein. An analyte sample or buffer was injected at a flow rate of 30 µl/min for 8 minutes, and washed for 20 minutes.

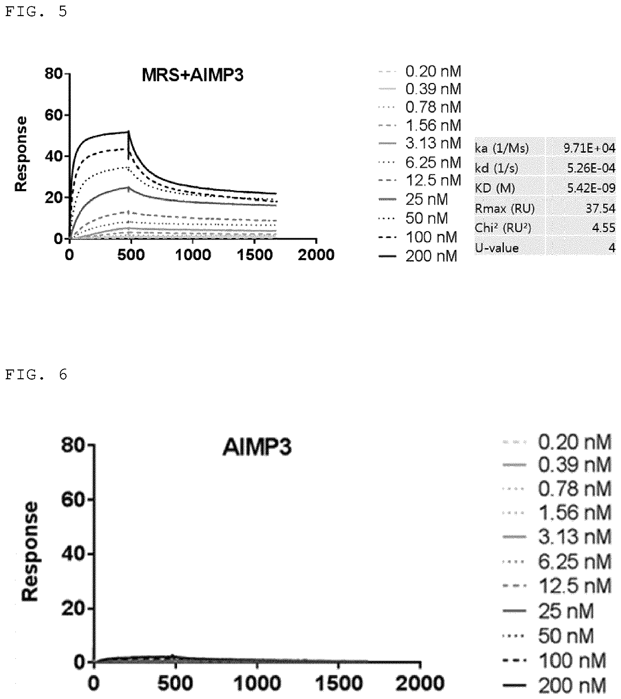

As a result, as shown in FIGS. 5 and 6, the 1E8 antibody bound to the MRS+AIMP3 protein but did not bind to the AIMP3 protein. It could also be verified that the 1E8 antibody had a KD value of 5.42 nM to MRS.

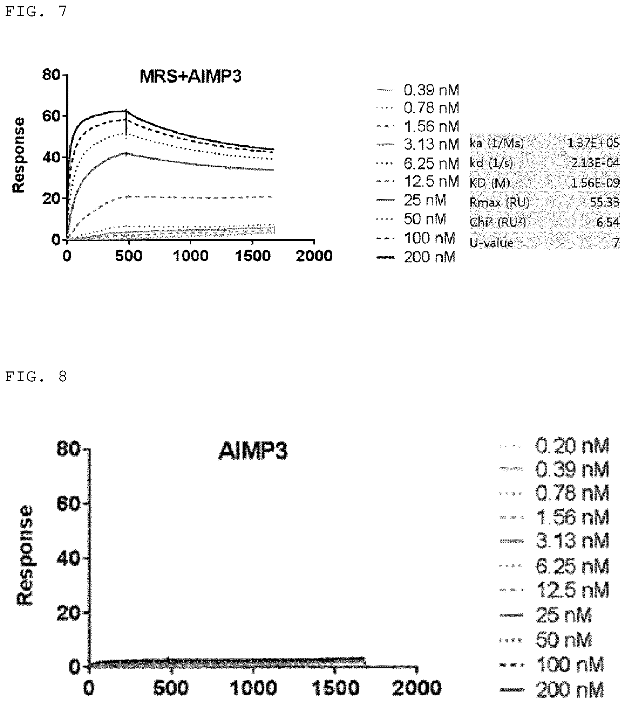

As shown in FIGS. 7 and 8, it could be verified that the 8A12 antibody bound to the MRS+AIMP3 protein but did not bind to the AIMP3 protein. It could also be verified that the 8A12 antibody had a KD value of 1.56 nM to MRS.

### 1-10. Verification on binding site of MRS antibodies

To investigate the binding site (epitope, main binding site) of the 1E8 or 8A12 antibody, the following test was conducted.

First, several MRS fragments with different lengths and loci in the MRS whole protein were constructed in consideration of GST, catalytic domain, and tRNA binding domain sites, and the MRS whole protein or each MRS fragment was cloned into the pcDNA3 vector (EV). The loci of the respective MRS fragments were selected at loci containing several small-unit domains, including the fragment of aa 1-266, the fragment of aa 267-597, the fragment of aa 1-598, the fragment of aa 598-900, the fragment of aa 660-860, the fragment of aa 660-900, the fragment of aa 730-900, and the like, in the whole amino acid sequence of MRS of SEQ ID NO: 1. In the fragments, the Myc protein was conjugated to the N-terminus of each peptide, and the Myc protein was used as a control. Then, 2 µg of the cloned vector DNA was transfected into H460 cells by using Turbofect (Thermo) according to the instruction of the manufacturer. After 24 h, the cells were harvested and western blotting was carried out. For the primary antibody, 1E8 and 8A12 antibodies were diluted at 1:5000 (0.2 µg/mL) before use.

Through the test, the epitope of the 1E8 and 8A12 antibodies was shown to be present in at least a region of aa 598-900 in the MRS protein of SEQ ID NO: 1.

Therefore, several small-unit domains, including the fragment of aa 811-840, the fragment of aa 821-850, the fragment of aa 831-860, the fragment of aa 841-870, the fragment of aa 846-875, the fragment of aa 851-880, the fragment of aa 856-885, the fragment of aa 861-890, the fragment of aa 866-895, and the fragment of aa 871-900, were constructed, and each peptide was coated at 300 ng/well on 96 well ELISA plates and ELISA was carried out according to the common protocol. The 1E8 or 8A12 antibody as a primary antibody was diluted to 10 nM (1x PBST-Tween 0.05%), and HRP-conjugated Goat anti-mouse IgG (Thermo) as a secondary antibody was diluted at 1:10000 (1xPBST-Tween0.05%). The absorbance was measured at 450 nm.

The test results verified that the 1E8 and 8A12 antibodies specifically recognized, as an epitope, the region of aa 861-900 (AQKADKNEVA AEVAKLLDLK KQLAVAEGKP PEAPKGKKKK, SEQ ID NO: 2) in the MRS protein. These test results indicate that other binding molecules (other antibodies and functional fragments thereof) recognizing the region of aa 861-900 as an epitope would also have excellent MRS-specific binding ability and MRS-identifying ability.

Hereinafter, an example employing antibodies with excellent MRS detection ability constructed above will be described for the novel pancreatic cancer examination method invented by the present inventors.

### 1-11. Pancreatic cancer cell staining using anti-MRS antibody of present disclosure and comparison of effect with commercially available antibody

As for the PANC-1 pancreatic cancer cell line and the SCK cell line (non-pancreatic cancer cell line), MRS fluorescence staining was carried out using 1E8 or 8A12 antibody. A commercially available MRS antibody (Abcam, Ab137105) was used as a control. Specifically, staining was carried out as follows. Each type of target cells prepared on slides was treated with PBS containing 0.2% Tween 20 to improve cell permeability thereof, and then blocked with 2% goat serum for 1 h. The slides were incubated with 1 µg/ml of the antibody (1E8 or 8A12 antibody, produced by Oncotag) of the present disclosure as a primary antibody or Ab137105 antibody (Abcam) at 37°C for 1 h, and washed three times with 0.05% TBST (500 µl). Thereafter, the Alexa-488-conjugated secondary antibody (purchased from Molecular Probes, Cat. No. A11001) as the fluorescent substance-conjugated secondary antibody was diluted to 1:100, and the slides were incubated with the secondary antibody at room temperature in the dark place, and washed three times with 0.05% TBST (500 µl). The tissue on the slides was treated with 20 µl of DAPI-added mounting solution (ProLong Gold antifade regent with DAPI/ Molecular Probes, Cat. No. P36931), and then the slides were covered with coverslips, followed by observation using a confocal laser microscope and a fluorescence microscope.

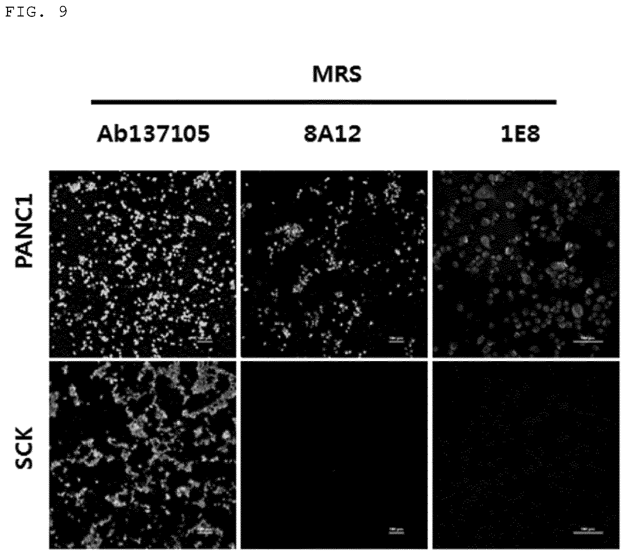

As shown in FIG. 9. the commercially available Ab137105 antibody, which has been identified to have poor MRS specificity in Example 1-7, non-specifically stained the PANC-1 pancreatic cancer cells and the SCK cells (non-pancreatic cancer cells), but the 1E8 and 8A12 antibodies of the present disclosure specifically stained only MRS.

In addition, the staining effects of the 1E8 and 8A12 antibodies of the present disclosure were investigated by utilizing the Thinprep device (Hologic. Inc) used in patient sample processing in clinical sites to manufacture Thinprep slides similar to clinical conditions of the PANC-1 cell line (see Example 2 below).

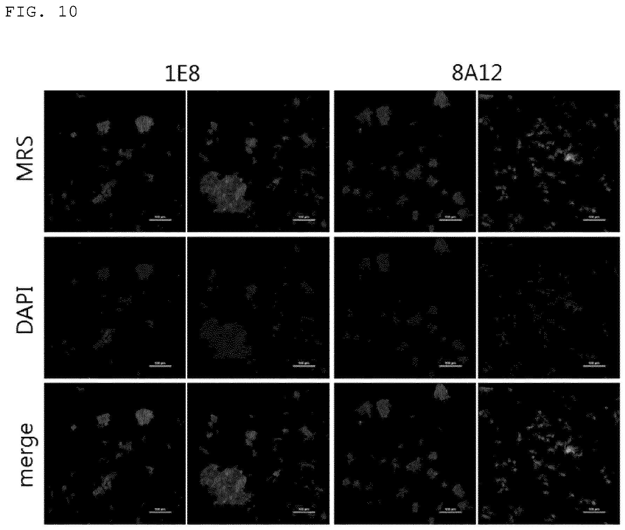

As a result, as shown in FIG. 10, the 1E8 and 8A12 antibodies of the present disclosure could be used together with a sample providing method (Thinprep slides or the like) that has been often used in the clinical sites.

### Example 2: Establishment and effect verification of pancreatic cancer cell-specific MRS expression detecting method (staining method) in cytodiagnosis

### Methods

1) Pancreatic cells as a specimen were obtained by endoscopic ultrasound fine needle aspiration (EUS-FNA). First, a linear array echoendoscope EUS (product name: GF-UCT140 or GF-UCT180, Olympus, Japan) was guided to the stomach or duodenum, and an ultrasound device mounted on the front end of the EUS was used to identify pancreatic masses through ultrasound imaging. The pancreatic cells were collected by allowing a needle for fine needle aspiration (product name: 22G Echo-ultraTM, Cook Medical, Cork, Ireland) to enter the ultrasound-guided masses.

Thereafter, the collected pancreatic cells were provided, as Cellient paraffin sections, on slides by a common method using the Cellient Automated Cell Block System (Hologic) (Antonio Ieni et al., Cell-block procedure in endoscopic ultrasound-guided-fine-needle-aspiration of gastrointestinal solid neoplastic lesions, World J Gastrointest Endosc 2015 August 25; 7(11): 1014-1022). Alternatively, the pancreatic cancer cells may be smeared or provided through direct smearing on ThinPrep slides by a common method using ThinPrep (Hologic.Inc) (de Luna R et al., Comparison of ThinPrep and conventional preparations in pancreatic fine-needle aspiration biopsy. Diagnostic Cytopathology, 2004 Feb;30(2):71-6). The cell samples each were subjected to the following examination method, and the results thereof were compared.

2) Pathological findings by a conventional cytological examination may be made by the staining results using H&E staining that has frequently been used up to now. The H&E staining was carried out using hematoxylin and eosin according to a common protocol (see protocol details in Example 3 below). Alternatively, the pathological findings may be made by the staining results through Pap staining. The Pap staining was carried out using hematoxylin, OG-6(Orange G-6), and eosin azure according to a common protocol (see protocol details in Example 3 below). The paraffin section samples were treated with staining substances after paraffin removal and hydration were carried out through common methods.

The cells were determined to be benign (normal) cells when: the cells were smeared in one layer on the slides; the nuclear to cytoplasmic ratio (N/C ratio) is small; and the nuclear membrane has a smooth shape. The cells were determined to be malignant cells when: the cells were three-dimensionally smeared; the nucleus/cytoplasm ratio was high; chromatin clumping appeared; the nuclear membrane had a rough shape; and nucleoli and mitosis appeared. The cells were identified as atypical cells when the cell change did not reach malignant cells but could not be diagnosed with benign.

3) The final clinical diagnosis results were made by doctors through a comprehensive final determination on the basis of the measurement results by imaging examinations (abdominal ultrasound, abdominal computed tomography, abdominal magnetic resonance imaging, endoscopic retrograde cholangiogram, and positron emission tomography) and pathological examinations (cytodiagnosis and biopsy).

4) The present inventors developed the immunohistochemistry (especially, immunofluorescence staining) for measuring the expression level of MRS in pancreatic cells and normal pancreatic cells (including benign pancreatitis cells but not cancer cells) as follows. Specifically, the paraffin sections were treated as follows.
① Paraffin removal: dissolve paraffin in oven at 60°C
② Hydration: wash with xylene for 5 min three times, wash with 100% ethanol two times, wash with 95% ethanol for 2 min, wash with 90% ethanol for 2 min, wash with 70% ethanol for 2 min, wash with D.W for 2 min, and wash with PBS for 5 min
③ Treatment for permeability increase: treat with 0.2% Triton X-100 for 30 min, and wash with PBS for 5min
④ Pretreatment: block with 2% goat serum for 1 h
⑤ Primary antibody treatment: dilute anti-MRS antibody (the 8A12 antibody of the present disclosure being representatively used, Oncotag) to 1 : 300, incubated with the diluted antibody at 4°C, and wash with PBS for 5 min three times
⑥ Color development: The Alexa-488-conjugated secondary antibody (purchased from Molecular Probes, Cat. No. A11001) as a secondary antibody to which a fluorescent substance is conjugated to 1:200 - 1:300, incubate with the secondary antibody at room temperature in the dark for 1 h, and wash with PBS for 5 min three times
⑦ Treat the tissue on the slides with 20 µl of DAPI-added mounting solution (ProLong Gold antifade regent with DAPI/ Molecular Probes, Cat. No. P36931), and cover the slides with coverslips.

The Thinprep slides were treated by a method comprising steps ③ to ⑦, without paraffin removal, and reference samples prepared by the method were observed by a confocal laser microscope and a fluorescence microscope.

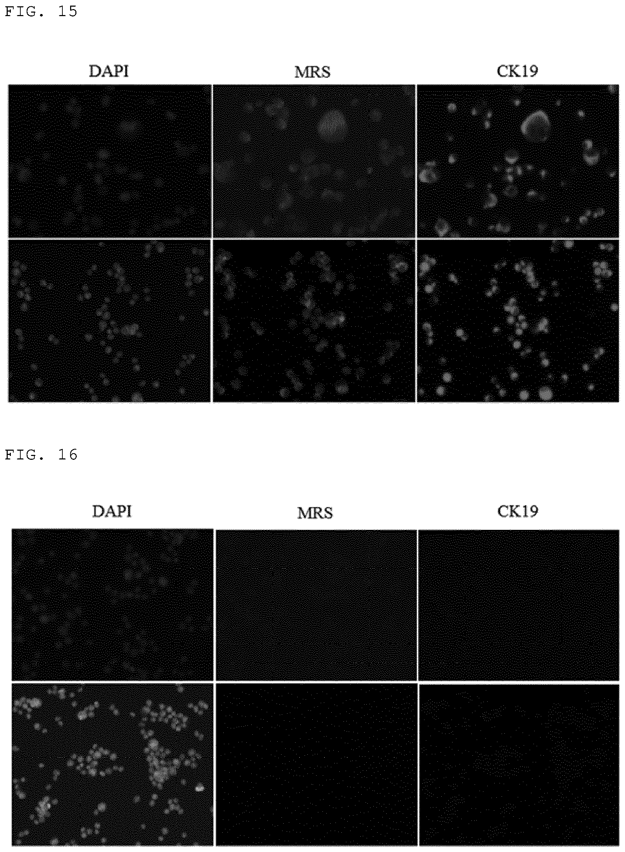

The MRS staining intensity was determined in the reference samples on the basis of the positive cell sample (PANC-1, see FIG. 15) and the negative cell sample (CT-26, see FIG. 16), and the cells showing a 2-fold or more increase compared with the negative cell control were determined to be pancreatic cancer cells. The diagnostic accuracy (sensitivity and specificity) was checked by comparing these results with the pathological findings and final clinical diagnosis results of the specimen.

### Results

Specific test results are shown in Tables 3, 4, and 5 below. As a result of applying the pancreatic cancer discrimination method through MRS staining of the present disclosure to 14 benign pancreatic cell samples and 94 pancreatic cancer (malignancy) cell samples, the conventional pathologic cytology results using H&E staining showed a sensitivity of about 75.5%, whereas the MRS staining of the present disclosure showed a sensitivity of 92.6%. In particular, even cell samples that have been classified as atypia through a conventional cytopathological examination could be discriminated for pancreatic cancer or non-pancreatic cancer, and thus the negative predictive value (NPV) through the conventional pathologic cytology is 36%, whereas the negative predictive value through MRS staining was significantly high. These results indicate that the staining method using MRS as a marker in pancreatic cancer in the present disclosure shows high discrimination ability (diagnostic ability) even at the cellular level, and as shown in Example 3 to be described later, the MRS used as a marker of the present disclosure was clearly distinguished from conventional commercially available pancreatic cancer markers (known pancreatic cancer markers, such as CEA) of which effectiveness had been difficult to exhibit in the diagnosis at the cellular level (that is, cytodiagnosis).

**[Table 3]**

| Comparative details: Comparison between conventional pathologic cytology results and MRS immunostaining results according to present disclosure, on the basis of final clinicopathological diagnosis results | | | |
|---|---|---|---|
| Conventional pathologic cytology | Final clinicopathological diagnosis | MRS immunostaining | |
| | | Positive | Negative |
| Malignancy (n=43) | Malignancy (n=43) | 42 | 1 |
| | Benign (n=0) | 0 | 0 |
| Suspicious of malignancy (n=29) | Malignancy (n=28) | 26 | 2 |
| | Benign (n=1) | 1 | 0 |
| Atypical (n=21) | Malignancy (n=18) | 17 | 1 |
| | Benign (n=3) | 2 | 1 |
| Negative for malignancy (n=15) | Malignancy (n=5) | 2 | 3 |
| | Benign (n=10) | 2 | 8 |

**[Table 4]**

| Comparative summarization: Comparison of conventional pathologic cytology results compared with final clinicopathological diagnosis results (the determinations of positive for malignancy and suspicious malignancy being classified as final positive and the determinations of atypia and negative for malignancy being classified as final negative in conventional pathologic cytology results on Table 3 above) | | | |
|---|---|---|---|
| | | Final clinicopathological diagnosis | |
| | | Malignancy | Benign |
| Conventional pathologic cytology | Positive | 71 | 1 |
| | Negative | 23 | 13 |
| Sensitivity: 75.5% Specificity: 92.9% Accuracy= 77.9% PPV: 98.6% NPV: 36.1% | | | |

| | | | |
|---|---|---|---|
| PPV: positive predictive value, NPV: negative predictive value | | | |

**[Table 5]**

| Comparative summarization: Comparison of MRS immunostaining results according to present disclosure compared with final clinicopathological diagnosis results | | | |
|---|---|---|---|
| | | Final clinicopathological diagnosis | |
| | | Malignancy | Benign |
| MRS immunostaining | Positive | 87 | 5 |
| | Negative | 7 | 9 |
| Sensitivity: 92.6% Specificity: 64.3% Accuracy= 88.1% PPV: 94.6% NPV: 56.3% | | | |

| | | | |
|---|---|---|---|
| PPV: positive predictive value, NPV: negative predictive value | | | |

### Example 3: Comparison of pancreatic cancer discrimination ability at the cellular level between commercially available pancreatic cancer marker CEA and present inventive MRS

### Methods

### 1) Patient groups and obtaining pancreatic cell samples for cytodiagnosis

The present study was performed using 26 cases of pancreatic cell samples collected and obtained from 26 patients with suspected pancreatic cancer through cell aspiration (fine needle aspiration) under endoscopic ultrasound, and approved by the Research Ethics Committee of the Gangnam Severance Hospital. The pancreatic cell samples were obtained from the patients by the same method as in Example 2 through endoscopic ultrasound fine needle aspiration (EUS-FNA). Thereafter, the collected pancreatic cells were prepared in a state of Cellient paraffin sections or Thinprep by a common method using the Cellient Automated Cell Block System (Hologic) (see Example 2).

The 26 patients with suspected pancreatic cancer were finally diagnosed with pancreatic cancer (13 cases) and normal pancreas (13 cases) through follow-up observation. Among 13 patients finally diagnosed with pancreatic cancer, seven cases were classified as having pancreatic cancer cells through histological observation by pathologists, and the other six cases were classified as having atypical cells by histological observation and finally diagnosed with pancreatic cancer through follow-up observation.

The patients provided written informed consent at the time of collection of pancreatic cells, and pancreatic cells, atypical cells, and normal cells were histologically confirmed by pathologists (see the standard for determination in Example 2). Respective types of representative diagnostic cases for normal cells, tumor cells and atypical cells among the 26 obtained samples are shown in the drawing for each test.

### 2) Conventional cytodiagnosis staining

H&E staining: The paraffin section samples were subjected to paraffin removal and hydration by treatment with xylene for 5 min three times, treatment with 100% ethanol for 2 min, treatment with 95% ethanol for 2 min, treatment with 90% ethanol for 2 min, treatment with 70% ethanol for 2 min, and treatment with tap water for 10 min. The samples were incubated with hematoxyline at room temperature for 30 s, and washed with tap water for 10 min. The samples were incubated with eosin at room temperature for 1 min, and washed with tap water for 10 min. The samples were subjected to dehydration and clearing by treatment with 70% ethanol for 1 min, 90% ethanol for 1 min, 95% ethanol for 1 min, 100% ethanol for 1 min, and xylene for 5 min three times. After the mounting solution was dropped on the tissue on the slides, the slides were covered with cover slides, and then the samples were observed under an optical microscope.

Pap staining: Papanicolaou staining was carried out using Varistain 24-4 stainer of Thermo Scientific according to the protocol embedded in the device. The protocol is shown in Table 6 below.

**[Table 6]**

| | reagent | program1 | program2 |
|---|---|---|---|
| 1 | water | 10 m | 10 m |
| 2 | Hema. | 1 m 30 s | 1 m 30 s |
| 3 | water | 30 s | 30 s |
| 4 | water | 30 s | 30 s |
| 5 | 0.5% Hcl. | 7 s | 7 s |
| 6 | 0.5% Amm. | 5 s | 5 s |
| 7 | water | 30 s | 30 s |
| 8 | 50% alc. | 30 s | 30 s |
| 9 | 70% alc. | 30 s | 30 s |
| 10 | 80% alc. | 30 s | 30 s |
| 11 | 95% alc. | 30 s | 30 s |
| 12 | OG 6 | 1 m | 10 s |
| 13 | 95% alc. | 30 s | 30 s |
| 14 | 95% alc. | 30 s | 30 s |
| 15 | EA 50 | 1 m | 10 s |
| 16 | 95% alc. | 30 s | 30 s |
| 17 | 95% alc. | 30 s | 30 s |
| 18 | 95% alc. | 30 s | 30 s |
| 19 | 99% alc. | 30 s | 30 s |
| 20 | 99%+xyl. | 20 s | 20 s |
| 21 | xyl. | 30 s | 30 s |
| 22 | xyl. | 30 s | 30 s |
| 23 | xyl. | 30 s | 30 s |
| 24 | xyl. | 30 s | 30 s |
| 25 | end. | | |

### 3) Criteria of determination in cytodiagnosis according to morphological pathology by conventional staining

The most critical evidence in the morphological diagnosis of malignancies is an invasive growth into surrounding normal tissues, but unlike the biopsy facilitating to prove the relationship between cancer and surrounding tissues, the cytodiagnosis cannot prove the relationship with the surrounding tissues since individual cells were extracted and smeared. Therefore, as an alternative, the atypia of individual cells is evaluated. The atypia refers to a high nuclear to cytoplasmic ratio (N/C ratio) resulting from cell nucleus enlargement and cytoplasm reduction, chromatin partially clumping without uniform distribution in the nucleus, appearance of nucleoli in the nucleus, appearance of mitosis, or the like. The diagnosis of malignancy can be made when all of these findings of atypia are shown and the degrees thereof are severe, but cells should be classified as atypical cells when the findings of the cells are partially shown or the degrees thereof are weak. The reason is that such findings may be partially shown even in benign lesions, such as severe inflammation. On the contrary, cells not showing any of the findings can be determined to be normal cells. It should be of course assumed that the cells can be observed at the site where the cells are collected.

### 4) Comparative staining using CEA, known commercially available pancreatic cancer marker, and present inventive MRS

Immunostaining for MRS was carried out in the same manner as described in Example 2. The immunostaining for MRS was carried out by the same procedures as in Example 2 except that Carcinoembryonic Antigen (CEA) antibody (purchased from Dako, Cat. No. M7072) was used for an optimal treatment condition.

### 5) Statistical analysis

The test results are expressed as mean ± standard deviation on the basis of three or more independent test results. For statistical significance, the Student's t test was used to analyze data to compare the difference among multiple groups. The test results were determined statistically significant when P-value was less than 0.05.

### Results

### 3-1. Immunostaining of normal cells

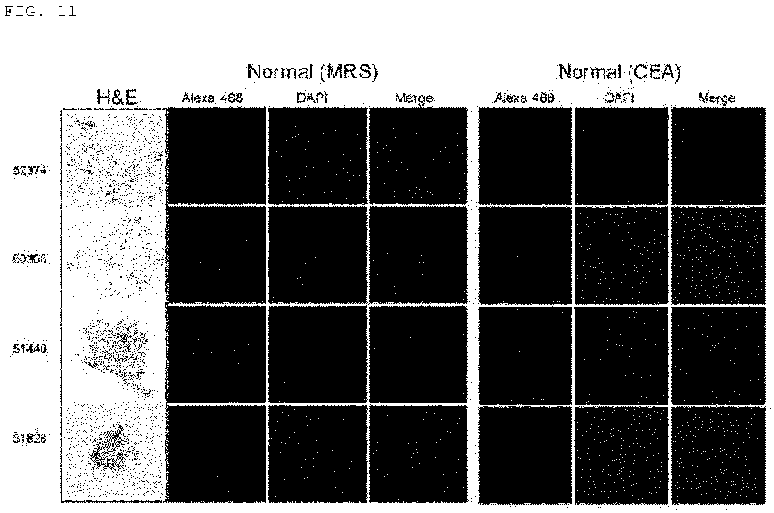

The immunofluorescence staining using MRS or CEA antibody was carried out on the cells that have been determined to be normal as a result of analysis of H&E staining of the cells isolated from the pancreas of normal patients or pancreatic cancer patients by EUS-FNA. The results are shown in FIG. 11.

As shown in FIG. 11, the pancreatic cells obtained from four patients showed no findings of tumor characteristics in H&E staining and thus were determined to be normal cells, and in the pancreatic cells determined to be normal, neither MRS nor CEA was stained.

### 3-2. Immunostaining of tumor cells

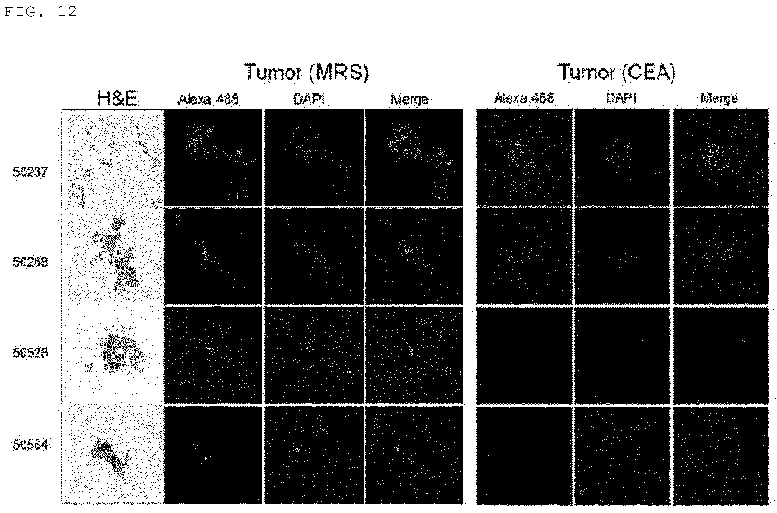

The immunofluorescence staining using MRS or CEA antibody was carried out on the cells that have been determined to be tumor cells as a result of analysis of H&E staining of the cells isolated from the pancreas of pancreatic cancer patients. The results are shown in FIG. 12.

As shown in FIG. 12, the pancreatic cells obtained from four patients were determined to be tumor cells in H&E staining. MRS was strongly stained in the tumor cells of all of four pancreatic cancer patients, indicating statistically significant results compared with the normal cell MRS staining group (p = 0.019) . However, CEA staining was weakly observed in only two patient samples, indicating no statistical significance compared with the normal cell MRS staining group (p = 0.187) .

### 3-3. Immunostaining of atypical cells

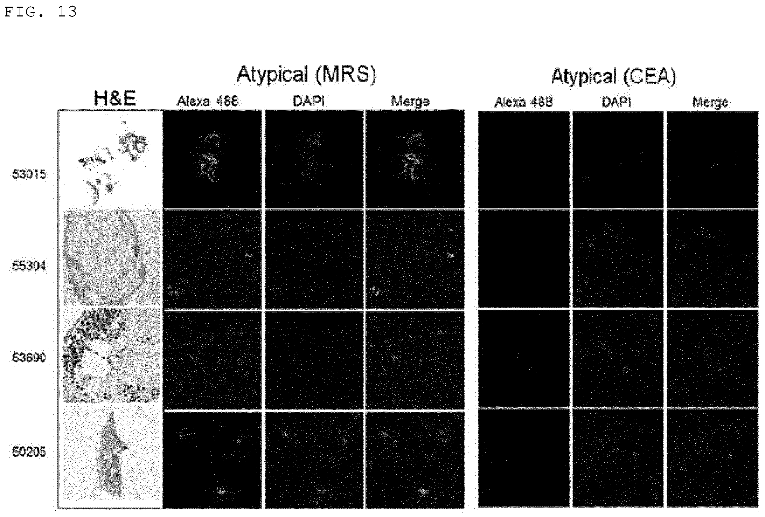

MRS or CEA staining was carried out on the pancreatic cells of patients, which are atypical cells that could not be clearly determined to be tumor cells by only H&E staining and have been finally determined to be tumor cells as a result of follow-up observation of the prognosis of the patients afterward. The results are shown in FIG. 13.

As shown in FIG. 13, it is not clearly discriminated whether the pancreatic cells classified as atypical cells through H&E staining are tumor cells or normal cells. Meanwhile, all the patients included in the atypical cell group were diagnosed with pancreatic cancer afterward. MRS was strongly stained in the tumor cells of all of four pancreatic cancer patients, indicating statistically significant results compared with the normal cell MRS staining group (p = 0.020). However, CEA staining was not observed in all of the four atypical pancreatic cells.

It can be seen from the results of Example 3 that performing both H&E staining and MRS staining on pancreatic cells isolated from patients with suspected pancreatic cancer can diagnose pancreatic cancer with much higher accuracy than other tumor markers. In addition, conventional tumor markers (e.g., CEA) cannot clearly discriminate whether the pancreatic cells, which cannot be determined to be tumor cells or normal cells even through H&E staining, are tumor cells or normal cells, but MRS is a very meaningful pancreatic cancer diagnostic marker in that MRS can clearly discriminate, with significantly high accuracy, whether the atypical cells are tumor cells.

### Example 4: Establishment of highly accurate pancreatic cancer discrimination method - MRS double staining

### 4-1. Finding cause of false-positive results

As shown in Example 3, it has been proved that MRS enables a diagnosis of pancreatic cancer with higher accuracy than conventional existing pancreatic cancer markers, such as CEA. However, the tests in the above-described examples were conducted on all the samples that have already been definitively determined, and therefore, it is necessary to investigate how accurately MRS can diagnose in a blind state after tissue was collected from patients suspected of developing pancreatic cancer. In addition, as shown in Example 2, MRS showed very excellent sensitivity in the diagnosis of pancreatic cancer, but MRS alone showed a somewhat low tendency of specificity as compared with sensitivity in the determination of pancreatic cancer. The present inventors, while testing the ability of MRS to discriminate pancreatic cancer in pancreatic samples obtained from new patients, observed that out of ten samples determined to be negative (non-pancreatic cancer) in pathologic cytology, three samples seemed to show MRS expression. That is, it was confirmed that the expression of MRS is high in some normal pancreatic cell samples, causing an error of false-positive results (a determination may be made diagnosing non-pancreatic cancer samples as a pancreatic cancer).

As a result of checking a cause of the false-positive determination by discriminate the overall specimens into cancer tissues and normal tissues through H&E staining and then carrying out MRS staining, it was verified as shown in FIG. 4 that MRS expression was high in normal acinar cells (cells present in the pancreatic parenchyma) in the normal pancreatic tissue.

### 4-2. Establishment of strategies for improving MRS diagnostic accuracy

As shown in the results in Example 4-1, a high level of MRS was expressed in even normal acinar cells, and thus MRS alone contributes to a false-positive rate in the determination of pancreatic cancer (i.e., the specificity of diagnosis is relatively low in the determination using MRS alone). Therefore, a strategy for reducing the false-positive rate was sought by excluding such acinar cells from the interpretation of the results.

After various trials, the present inventors first established that pancreatic cancer (especially pancreatic ductal cancer) can be discriminated with very high accuracy at the cellular level by using, as a double marker, MRS and cytokeratin 19 (CK19) among several subsidiary marker candidates. Therefore, double staining using MRS and CK19 was invented as follows.

### 4-3. Establishment of double staining

As for paraffin section samples, paraffin was melted in an oven at 60°C, and subjected to paraffin removal and hydration by washing with xylene for 5 min three times, washing with 100% ethanol two times, washing with 95% ethanol for 2 min, washing with 90% ethanol for 2 min, washing with 70% ethanol for 2 min, and washing with D.W for 2 min.

For double staining of MSR and CK19, a specific test protocol was established such that MRS was detected in green (Alexa Fluor 488) and CK19 was detected in red (Texas Red). First, cells were treated with 0.2% Triton X-100 to increase cell permeability. An incubation was conducted with 2% goat serum, a blocking solution, for 1 h. Thereafter, the pancreatic cells (specimen) was incubated with the primary antibody, methionyl-tRNA synthetase antibody (1 : 200) and anti-CK19 antibody(1 : 100) at 4°C overnight. Thereafter, the cells were washed three times or more by performing at least 30 times of dipping in 1× TBST (assumed as one time), and then incubated with Alexa Fluor 488- and Texas Red-conjugated secondary antibodies (1:200 to 1:300, ThermoFisher Scientific, catalog no. A-11001/ SANTA CRUZ BIOTECHNOLOGY, INC., catalog no. sc-278) at room temperature in a dark place for 1 h. The cells were washed three times or more by performing at least 30 times of dipping in 1× TBST (assumed as one time). Thereafter, the DAPI-added mounting solution (ProLong Gold antifade regent with DAPI, Molecular probes, Cat. No. P36931) was dropped onto the tissue on slides, and then the cells were covered with cover slides, and the samples were observed by a fluorescent microscope.

The standard for pancreatic cancer discrimination is as shown in Table 7 below. The cells were determined to be pancreatic cancer cells when high levels of MRS and CK19 were detected (increasingly expressed). That is, the cells were classified as Positive for MRS (+) and CK19 (+) and classified as Negative for the others.

**[Table 7]**

| MRS | CK19 | Readout |
|---|---|---|
| + | + | cancer |
| + | - | acinar cell |
| - | + | ductal cell |
| - | - | fibrotic cell or others |

When the level of MRS in a specimen sample was increased by 2-fold or more as compared with the negative cell sample (CT-26), together with expression of CK19, the cells were determined to be pancreatic cancer cells. The intensity of staining was based on those of positive cell sample (PANC-1) and the negative cell sample (CT-26).

FIG. 15 shows staining patterns (staining intensity of each marker) of MRS and CK19 which were used for the staining of pancreatic cancer cells (PANC-1 cell line) that were cultured for positive control setup, in the discrimination of pancreatic cancer through cytodiagnosis by double staining of the present disclosure. It was verified that both MRS and CK19 were detected with high intensity in pancreatic cancer cells (that is, the expression of each marker was significantly increased).

FIG. 16 shows staining patterns (staining intensity of each marker) of MRS and CK19 which were used for the staining of non-pancreatic cancer cells (CT26 cell line) that were cultured for negative control setup, in the discrimination of pancreatic cancer through cytodiagnosis by double staining of the present disclosure. MRS and CK19 were not substantially detected in the non-pancreatic cancer cells.

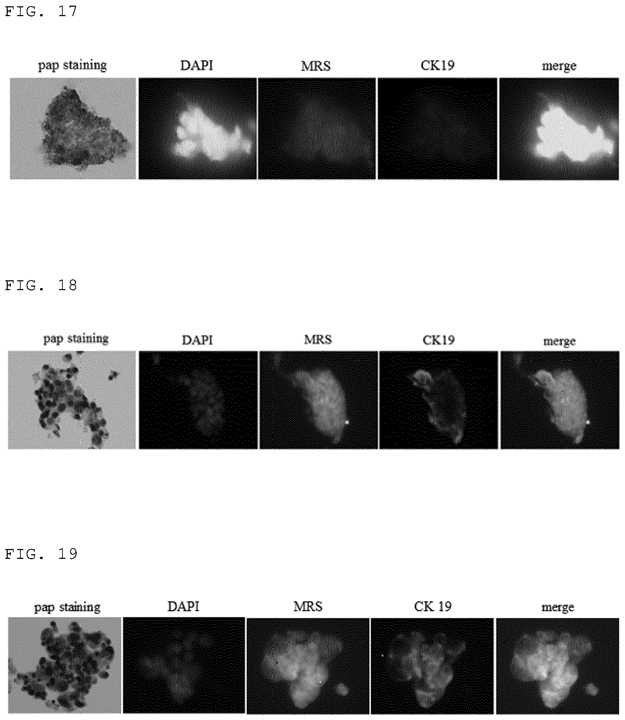

FIG. 17 shows staining intensity of each marker when the pancreatic acinar cell sample collected from a patient was stained with MRS and CK19 in order to investigate staining patterns on the acinar cells in cytodiagnosis by double staining of the present disclosure. In the acinar cells, only the green signal was strongly shown since the detection intensity of only MRS was high.

### Example 5: Evaluation on pancreatic cancer discrimination ability of present inventive double staining in cytodiagnosis

The discrimination of pancreatic cancer was carried out by applying double staining of the present disclosure to 29 new pancreatic cancer cell specimens obtained from patients different from those used in the above-described examples. These pancreatic cancer cells were collected by EUS-FNA in the same manner as described above. The double staining of the present disclosure was carried out while pathological diagnosis results or final clinical diagnosis results were blinded.

### 5-1. Staining pattern in patient classified as atypical cells by conventional pathologic cytology and thus undiagnosable but finally diagnosed with pancreatic cancer

In the cell sample that has been classified as atypical cells by conventional pathologic cytology through pap staining and diagnosed with malignancy in the final clinical diagnosis, the double staining of the present disclosure was applied to discriminate whether it is pancreatic cancer or not.

As shown in FIG. 18, as a result of double staining of the present disclosure, both MRS (green) and CK19 (red) showed high levels of detection intensity, and thus the cell sample could be determined to be malignant tumor cells (i.e., pancreatic cancer). The atypical cells identified by conventional pathologic cytology are undiagnosable, thereby requiring re-examination. The staining method of the present disclosure was confirmed to reduce the risk of re-examination and to be very useful in the discrimination (determination) of atypical cells into pancreatic cancer cells or benign cells.

### 5-2. Staining pattern in patient undiagnosed due to being suspicious of pancreatic cancer by conventional pathologic cytology but finally diagnosed with pancreatic cancer

In the cell sample that has not been diagnosed with pancreatic cancer by conventional pathologic cytology through pap staining and thus temporarily diagnosed in a state of suspicious of pancreatic cancer (malignancy) but diagnosed with malignant tumor cells in the final clinical diagnosis, the double staining of the present disclosure was applied to discriminate between pancreatic cancer cells and benign cells.

As shown in FIG. 19, as a result of double staining of the present disclosure, both MRS (green) and CK19 (red) showed high levels of detection intensity, and thus the cell sample could be determined to be malignant tumor cells (i.e., pancreatic cancer). Therefore, the staining method of the present disclosure was again confirmed to reduce the risk of re-examination and to be very useful in the discrimination (determination) of pancreatic cancer in cases of undiagnosed cells.

### 5-3. Staining pattern in the specimen from patient diagnosed with pancreatic cancer by conventional pathologic cytology and finally diagnosed with pancreatic cancer

In the cell sample that has been identified as atypical cells as a result of analysis by conventional pathological examinations through pap staining and diagnosed with malignant tumor cells in the final clinical diagnosis, the double staining of the present disclosure was applied to discriminate between pancreatic cancer cells and benign cells.

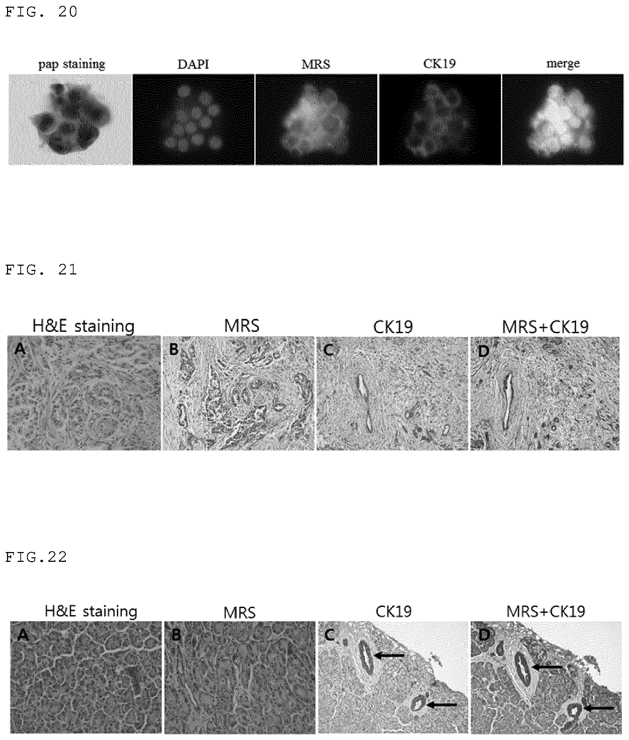

As shown in FIG. 20, both MRS (green) and CK19 (red) showed high levels of detection intensity, and thus the cell sample could be determined to be malignant tumor cells (i.e., pancreatic cancer).

### 5-4. Overall result

A comparison was made among the determination results obtained by carrying out the double staining of the present disclosure, the final clinicopathological diagnosis results, and the pathologic cytology diagnosis results, for each cell specimen, and the comparison results are shown in Table 8. As a result of double staining of the present disclosure, the cell specimen was classified as Positive of pancreatic cancer for MRS (+) and CK19 (+) and Negative of pancreatic cancer for the other case (i.e., either MRS or CK19 being stained, or MRS (-) and CK19 (-))

**[Table 8]**

| | | | |
|---|---|---|---|
| Comparison of examination results through present inventive MRS and CK19 double staining compared with final clinicopathological diagnosis results | | | |

| | | Final clinicopathological diagnosis | |
|---|---|---|---|
| | | Malignancy | Benign |
| MRS+CK19 immunostaining | Positive | 28 | 0 |
| | Negative | 0 | 1 |
| Sensitivity: 100% Specificity: 100% Accuracy= 100% PPV: 100% NPV: 100% | | | |

| | | | |
|---|---|---|---|
| PPV: positive predictive value, NPV: negative predictive value | | | |

As shown in Table 8 above, the determination of pancreatic cancer through MRS and CK19 double staining of the present disclosure showed a sensitivity of 100%, a specificity of 100%, a positive predictive value (PPV) of 100%, and a negative predictive value (NPV) of 100%, indicating an improvement in diagnostic accuracy.

As shown in Example 5, the double staining of the present disclosure can clearly discriminate between malignant tumor cells and non-tumor cells from the cells that have been undiagnosable and undiagnosed by a conventional method, including atypical cells, thereby significantly improving the diagnostic efficiency in cytodiagnosis.

### Example 6: Evaluation on pancreatic cancer discrimination ability of present inventive double staining in surgery biopsy

The double staining of the present disclosure was applied to clinical tissues containing both pancreatic cancer and surrounding normal pancreas area. The tissues obtained by surgery were paraffin-embedded by a common method, and then sliced. The double staining and determination results according to the present disclosure are shown in Table 9 below. As shown in Table 9 below, the tissue samples, unlike cytodiagnosis samples, were discriminated into acinar and pancreatic duct in the pancreatic tissues and thus can be separately determined according to the region.

**[Table 9]**

| Final clinicopathologi cal diagnosis | | Number of specim ens | Present inventive double staining | | | |
|---|---|---|---|---|---|---|
| | | | MRS (+) CK19 (+ ) | MRS (+) CK19 (- ) | MRS (-) CK19 (+ ) | MRS (-) CK19 (- ) |
| Normal pancre | acinar cell | 10 | 0 | 10 | 0 | 0 |
| as | Pancreat ic ductal cell | | 0 | 0 | 10 | 0 |
| Pancreatic cancer | | 10 | 10 | 0 | 0 | 0 |

As shown in Table 9 above, the test results verified that the accuracy in discrimination between pancreatic cancer and normal pancreas through the double staining of the present disclosure reached 100% (sensitivity 100%, specificity 100%). It was especially verified that the double staining of the present disclosure can be used to provide information about normal acinar cells, thereby significantly increasing the diagnostic accuracy.

FIGS. 21 and 22 show respective types of representative diagnostic cases for pancreatic tissue specimens. FIG. 21 shows staining patterns of the pancreatic tissue, which was determined to be a pancreatic cancer tissue by pathological findings through H&E staining, wherein MRS was highly expressed in the pancreatic cancer tissue by staining of MRS alone; CK19 was expressed in the pancreatic cancer tissue by staining of CK19 alone; and finally, both MRS and CK19 were detected at high levels in co-staining (the double staining of the present disclosure).

FIG. 22 shows staining patterns of the normal tissue, which was determined to be a normal pancreatic tissue through H&E staining, wherein both acinar cells and pancreatic ductal cells were verified to be present together. Only the acinar cells were stained by staining of MRS alone, and CK19 was highly detected and observed in red (arrows) in only the pancreatic ductal cells by staining of CK19 alone. Finally, in the co-staining (the double staining of the present disclosure), MRS was highly detected in the acinar cells, and CK19 were highly detected and observed in red (arrows) in pancreatic ductal cells.

### Industrial Applicability

As set forth above, the present disclosure relates to a method for diagnosis of pancreatic cancer by using methionyl-tRNA synthetase (MRS). Methionyl-tRNA synthetase (MRS) as a pancreatic cancer marker shows a much higher diagnostic accuracy than a conventional pancreatic cancer marker, such as CEA, so that a clear determination of pancreatic cancer can be made by analyzing the expression or non-expression of methionyl-tRNA synthetase (MRS) in pancreatic cells, and an equivalent effect is achieved in even the cells identified as atypical cells by ordinary staining, and thus MRS can be very helpfully used in the diagnosis of pancreatic cancer and thus has high industrial applicability in the field of in-vitro diagnostic industry.

## Claims

1. An in vitro method for diagnosis of pancreatic cancer, the method comprising:
(a) measuring the expression level of methionyl-tRNA synthetase (MRS) protein in a sample of pancreatic cells collected from a latent patient; and detecting cytokeratin 19 (CK19) protein in said sample;
(b) comparing the expression level of the methionyl-tRNA synthetase protein measured in step (a) with that of a negative control, and determining the latent patient to be a pancreatic cancer patient when cytokeratin (CK19) protein is detected and the expression level of the methionyl-tRNA synthetase protein is increased compared with that of the negative control.

2. The in vitro method of claim 1, wherein the pancreatic cells were isolated by fine needle aspiration.

3. The in vitro method of claim 1 or 2, wherein the method further comprises the following steps before, simultaneously with, or after the measuring of the expression level of the MRS protein:
(i) staining the pancreatic cells collected from the latent patient with: at least one nuclei-staining solution selected from the group consisting of 4',6-diamidino-2-phenylindole (DAPI), methylene blue, acetocarmine, toluidine blue, hematoxylin, and Hoechst; and at least one cytoplasm-staining solution selected from the group consisting of eosin, crystal violet, and Orange G; and
(ii) identifying the pancreatic cells as malignant tumor cells, atypical cells, or normal cells, through the staining of the pancreatic cells.

4. The in vitro method of claim 3, wherein in step (ii), on the basis of results of the staining in step (i) :
the pancreatic cells are identified as malignant tumor cells when the pancreatic cells show two or more types of morphological abnormality selected from the group consisting of: a three-dimensional smear of cells; a high nuclear to cytoplasmic ratio (N/C ratio); an appearance of chromatin clumping; a rough-shaped nuclear membrane; an appearance of nucleoli, and an appearance of mitosis;
the pancreatic cells are identified as normal cells when the pancreatic cells are smeared in one layer; the nuclear to cytoplasmic ratio (N/C ratio) is small; and the nuclear membrane has a smooth shape; and
the pancreatic cells are identified as atypical cells when the pancreatic cells have neither a cell change leading to malignant tumor cells, nor can be determined to be normal.

5. The in vitro method of claim 1, wherein the method comprises determining the pancreatic cells as pancreatic cancer cells when the CK19 protein is expressed and the expression level of the MRS protein is increased compared with that of the negative control; and/or wherein the expression level of the protein is measured using any one of western blotting, enzyme-linked immunosorbent assay (ELISA), radioimmunoassay, radioimmunodiffusion, Ouchterlony immunodiffusion, rocket immunoelectrophoresis, immunostaining, immunoprecipitation assay, complement fixation assay, fluorescence activated cell sorter (FACS) assay, surface plasmon resonance (SPR) assay, or protein chip assay.

6. An in vitro use of a composition for diagnosis of pancreatic cancer, the composition comprising an agent for measuring the expression level of a methionyl-tRNA synthetase (MRS) protein and an agent for measuring the expression level of a cytokeratin 19 (CK19) , wherein the agents are antibodies or aptamers specifically binding to the MRS and CK19 proteins, wherein the sample comprises pancreatic cells collected from a latent patient.

7. The in vitro use of the composition of claim 6, wherein the methionyl-tRNA synthetase protein comprises an amino acid sequence defined by SEQ ID NO: 1.

8. The *in vitro* use of the composition of claim 7, wherein the antibodies are antibodies or functional fragments thereof which specifically bind to an epitope region comprising an amino acid sequence defined by SEQ ID NO: 2 in the MRS protein.

9. The in vitro use of the_composition of claim 8, wherein the antibodies comprise,
a light chain variable region (VL) comprising an amino acid sequence defined by SEQ ID NO: 28 and a heavy chain variable region (VH) comprising an amino acid sequence defined by SEQ ID NO: 30, or
a light chain variable region (VL) comprising an amino acid sequence defined by SEQ ID N
O: 32 and a heavy chain variable region (VH) comprising an amino acid sequence defined by SEQ ID NO: 34.

10. Use of a kit in the *in vitro* method for diagnosis of pancreatic cancer according to claim 1, the kit comprising agents for measuring of the expression levels of methionyl-tRNA synthetase (MRS) protein and cytokeratin 19 (CK19) protein, wherein the agents are antibodies or aptamers specifically binding to the MRS and CK19 proteins.

11. The in vitro method of claim 1, wherein the method has a sensitivity of 80% or higher and a specificity of 80% or higher.

## Patentansprüche

1. In-vitro-Verfahren zum Diagnostizieren von Pankreaskrebs, wobei das Verfahren umfasst:
(a) Messen eines Expressionsniveaus von Methionyl-tRNA-Synthetase(MRS)-Protein in einer Probe von Pankreaszellen, die einem latenten Patienten entnommen wurden; und Nachweisen von Cytokeratin-19(CK19)-Protein in der Probe;
(b) Vergleichen des in Schritt (a) gemessenen Expressionsniveaus des Methionyl-tRNA-Synthetaseproteins mit dem einer negativen Kontrolle und Bestimmen, dass der latente Patient ein Pankreaskrebspatient ist, wenn Cytokeratin-19(CK19)-Protein nachgewiesen wird und das Expressionsniveau des Methionyl-tRNA-Synthetaseproteins im Vergleich zu der negativen Kontrolle erhöht ist.

2. In-vitro-Verfahren nach Anspruch 1, wobei die Pankreaszellen durch Feinnadelaspiration isoliert wurden.

3. In-vitro-Verfahren nach Anspruch 1 oder 2, wobei das Verfahren ferner die folgenden Schritte vor, gleichzeitig mit oder nach dem Messen des Expressionsniveaus des MRS-Proteins umfasst:
(i) Anfärben der dem latenten Patienten entnommenen Pankreaszellen mit: mindestens einer zellkernanfärbenden Lösung, die ausgewählt ist aus der Gruppe bestehend aus 4',6-Diamidino-2-phenylindol (DARI), Methylen-blau, Acetocarmin, Toluidin-blau, Hämatoxylin und Hoechst; und mindestens einer zytoplasmaanfärbenden Lösung, die ausgewählt ist aus der Gruppe bestehend aus Eosin, Kristallviolett und Orange G; und
(ii) Identifizieren der Pankreaszellen als bösartige Tumorzellen, atypische Zellen oder normale Zellen durch das Anfärben der Pankreaszellen.

4. In-vitro-Verfahren nach Anspruch 3, wobei in Schritt (ii) auf der Basis von Ergebnissen des Anfärbens in Schritt (i) :
die Pankreaszellen als bösartige Tumorzellen identifiziert werden, wenn die Pankreaszellen zwei oder mehr Arten einer morphologischen Anomalie zeigen, die ausgewählt sind aus der Gruppe bestehend aus: einem dreidimensionalen Zellabstrich; einem hohen Verhältnis von Zellkern zu Zytoplasma (N/C-Verhältnis); einer sich zeigenden Chromatinverklumpung; einer unregelmäßig geformten Zellkernmembran, sich zeigenden Nucleoli und sich zeigender Mitose;
die Pankreaszellen als normale Zellen identifiziert werden, wenn ein Abstrich der Pankreaszellen eine einzige Schicht bildet; das Verhältnis von Zellkern zu Zytoplasma (N/C-Verhältnis) niedrig ist; und die Zellkernmembran eine glatte Form aufweist; und
die Pankreaszellen als atypische Zellen identifiziert werden, wenn die Pankreaszellen weder eine Zelländerung aufweisen, die zu bösartigen Tumorzellen führt, noch als normal bestimmt werden können.

5. In-vitro-Verfahren nach Anspruch 1, wobei das Verfahren ein Bestimmen der Pankreaszellen als Pankreaskrebszellen umfasst, wenn das CK19-Protein exprimiert wird und das Expressionsniveau des MRS-Proteins im Vergleich zu dem der negativen Kontrolle erhöht ist; und/oder wobei das Expressionsniveau des Proteins unter Verwendung von irgendeinem von Western-Blotting, Enzyme-linked Immunosorbent Assay (ELISA), Radioimmun-Assay, Radioimmundiffusion, Ouchterlony-Immundiffusion, Rocket-Immunelektrophorese, Immunostaining, Immunpräzipitations-Assay, Komplementbindungs-Assay, Fluoreszenzaktivierungs-Zellsortierungs(FACS)-Assay, Oberflächenplasmonenresonanz(SPR)-Assay oder Proteinchip-Assay gemessen wird.

6. In-vitro-Verwendung einer Zusammensetzung für eine Diagnose von Pankreaskrebs, wobei die Zusammensetzung ein Mittel zum Messen des Expressionsniveaus eines Methionyl-tRNA-Synthetase(MRS)-Proteins und ein Mittel zum Messen des Expressionsniveaus eines Cytokeratin-19(CK19)-Proteins umfasst, wobei die Mittel Antikörper oder Aptamere sind, die sich spezifisch an die MRS- und CK19-Proteine binden, wobei die Probe Pankreaszellen umfasst, die einem latenten Patienten entnommen wurden.

7. In-vitro-Verwendung der Zusammensetzung nach Anspruch 6, wobei das Methionyl-tRNA-Synthetaseprotein eine Aminosäuresequenz umfasst, die definiert wird durch SEQ ID NO: 1.

8. In-vitro-Verwendung der Zusammensetzung nach Anspruch 7, wobei die Antikörper Antikörper oder funktionelle Fragmente davon sind, die sich spezifisch an eine Epitopregion in dem MRS-Protein binden, die eine Aminosäuresequenz umfasst, die definiert wird durch SEQ ID NO: 2.

9. In-vitro-Verwendung der Zusammensetzung nach Anspruch 8, wobei die Antikörper umfassen:
eine variable Region einer leichten Kette (VL), die eine Aminosäuresequenz umfasst, die definiert wird durch SEQ ID NO: 28, und eine variable Region einer schweren Kette (VH), die eine Aminosäuresequenz umfasst, die definiert wird durch SEQ ID NO: 30, oder
eine variable Region einer leichten Kette (VL), die eine Aminosäuresequenz umfasst, die definiert wird durch SEQ ID NO: 32, und eine variable Region einer schweren Kette (VH), die eine Aminosäuresequenz umfasst, die definiert wird durch SEQ ID NO: 34.

10. Verwendung eines Kits in dem In-vitro-Verfahren zum Diagnostizieren von Pankreaskrebs nach Anspruch 1, wobei das Kit Mittel zum Messen der Expressionsniveaus von Methionyl-tRNA-Synthetase(MRS)-Protein und Cytokeratin-19(CK19)-Protein umfasst, wobei die Mittel Antikörper oder Aptamere sind, die sich spezifisch an die MRS- und CK19-Proteine binden.

11. In-vitro-Verfahren nach Anspruch 1, wobei das Verfahren eine Sensitivität von 80 % oder mehr und eine Spezifität von 80 % oder mehr aufweist.

## Revendications

1. Méthode in vitro de diagnostic du cancer du pancréas, la méthode comprenant :
(a) la mesure du niveau d'expression de la protéine de méthionyl-ARNt synthétase (MRS) dans un échantillon de cellules pancréatiques collectées auprès d'un patient latent ; et la détection de la protéine de cytokératine 19 (CK19) dans ledit échantillon ;
(b) la comparaison du niveau d'expression de la protéine de méthionyl-ARNt synthétase mesuré dans l'étape (a) avec celui d'un contrôle négatif, et la détermination du fait que le patient latent est un patient atteint d'un cancer du pancréas lorsque la protéine de cytokératine 19 (CK19) est détectée et que le niveau d'expression de la protéine de méthionyl-ARNt synthétase est supérieur à celui du contrôle négatif.

2. Méthode in vitro selon la revendication 1, dans laquelle les cellules pancréatiques ont été isolées par aspiration par une aiguille fine.

3. Méthode in vitro selon la revendication 1 ou 2, dans laquelle la méthode comprend en outre les étapes suivantes avant, simultanément avec ou après la mesure du niveau d'expression de la protéine MRS :
(i) la coloration des cellules pancréatiques collectées auprès du patient latent avec : au moins une solution colorant les noyaux choisie dans le groupe constitué par le 4',6-diamidino-2-phénylindole (DAPI), le bleu de méthylène, l'acétocarmine, le bleu de toluidine, l'hématoxyline et un colorant Hoechst ; et au moins une solution colorant le cytoplasme choisie dans le groupe constitué par l'éosine, le violet de cristal et l'Orange G ; et
(ii) l'identification des cellules pancréatiques en tant que cellules tumorales malignes, cellules atypiques ou cellules normales, au moyen de la coloration des cellules pancréatiques.

4. Méthode in vitro selon la revendication 3, dans laquelle dans l'étape (ii), à partir des résultats de la coloration de l'étape (i) :
les cellules pancréatiques sont identifiées comme des cellules tumorales malignes lorsque les cellules pancréatiques présentent deux types d'anomalie morphologique ou plus choisis dans le groupe constitué par : un étalement de cellules tridimensionnel ; un rapport nucléaire à cytoplasmique (rapport N/C) élevé ; une apparence d'agglomération de la chromatine ; une membrane nucléaire de forme rugueuse ; une apparence de nucléoles, et une apparence de mitose ;
les cellules pancréatiques sont identifiées comme des cellules normales lorsque les cellules pancréatiques sont étalées en une seule couche ; le rapport nucléaire à cytoplasmique (rapport N/C) est faible ; et la membrane nucléaire a une forme lisse ; et
les cellules pancréatiques sont identifiées comme des cellules atypiques lorsque les cellules pancréatiques ni ne présentent un changement cellulaire menant à des cellules tumorales malignes, ni ne peuvent être déterminées comme étant normales.

5. Méthode in vitro selon la revendication 1, dans laquelle la méthode comprend la détermination des cellules pancréatiques comme cellules cancéreuses pancréatiques lorsque la protéine CK19 est exprimée et que le niveau d'expression de la protéine MRS est supérieur à celui du contrôle négatif ; et/ou lorsque le niveau d'expression de la protéine est mesuré en utilisant l'un quelconque du Western blot, du dosage d'immunoabsorption par enzyme liée (ELISA), du radioimmunodosage, de la radioimmunodiffusion, de l'immunodiffusion d'Ouchterlony, de l'immunoélectrophorèse en fusée, de l'immunocoloration, du dosage par immunoprécipitation, du dosage par fixation du complément, du dosage par tri cellulaire activé par fluorescence (FACS), du dosage par résonance de plasmons de surface (SPR) ou du dosage sur puce à protéines.

6. Utilisation in vitro d'une composition pour le diagnostic d'un cancer du pancréas, la composition comprenant un agent pour la mesure du niveau d'expression d'une protéine de méthionyl-ARNt synthétase (MRS) et un agent pour la mesure du niveau d'expression d'une cytokératine 19 (CK19), dans laquelle les agents sont des anticorps ou des aptamères se liant spécifiquement aux protéines MRS et CK19, dans laquelle l'échantillon comprend des cellules pancréatiques collectées auprès d'un patient latent.

7. Utilisation in vitro de la composition selon la revendication 6, dans laquelle la protéine de méthionyl-ARNt synthétase comprend une séquence d'acides aminés définie par la SEQ ID NO : 1.

8. Utilisation *in vitro* de la composition selon la revendication 7, dans laquelle les anticorps sont des anticorps ou des fragments fonctionnels de ceux-ci qui se lient spécifiquement à une région d'épitope comprenant une séquence d'acides aminés définie par la SEQ ID NO : 2 dans la protéine MRS.

9. Utilisation in vitro de la composition selon la revendication 8, dans laquelle les anticorps comprennent
une région variable de chaîne légère (VL) comprenant une séquence d'acides aminés définie par la SEQ ID NO : 28 et une région variable de chaîne lourde (VH) comprenant une séquence d'acides aminés définie par la SEQ ID NO : 30, ou
une région variable de chaîne légère (VL) comprenant une séquence d'acides aminés définie par la SEQ ID NO : 32 et une région variable de chaîne lourde (VH) comprenant une séquence d'acides aminés définie par la SEQ ID NO : 34.

10. Utilisation d'un kit dans la méthode *in vitro* de diagnostic d'un cancer du pancréas selon la revendication 1, le kit comprenant des agents pour la mesure des niveaux d'expression de la protéine de méthionyl-ARNt synthétase (MRS) et de la protéine de cytokératine 19 (CK19), dans laquelle les agents sont des anticorps ou des aptamères se liant spécifiquement aux protéines MRS et CK19.

11. Méthode in vitro selon la revendication 1, dans laquelle la méthode a une sensibilité supérieure ou égale à 80 % et une spécificité supérieure ou égale à 80 %.


### Sequence Listing

<110> MEDICINAL BIOCONVERGENCE RESEARCH CENTER INDUSTRY-ACADEMIC COOPERATION FOUNDATION, YONSEI UNIVERSITY
<120> Method for diagnosing pancreatic cancer using methionyl-tRNA synthetase, and kit for diagnosing pancreatic cancer using thereof
<130> OP18-0148
<150> KR 10-2017-0113255
   <151> 2017-09-05
<160> 72
<170> KoPatentIn 3.0
<210> 1
   <211> 900
   <212> PRT
   <213> Artificial Sequence
<220>
   <223> Amino acid sequence of MRS(methionyl-tRNA synthetase)
<400> 1
<210> 2
   <211> 40
   <212> PRT
   <213> Artificial Sequence
<220>
   <223> antibody epitope(main binding site)
<400> 2
   Ala Gln Lys Ala Asp Lys Asn Glu Val Ala Ala Glu Val Ala Lys Leu
   1 5 10 15
   Leu Asp Leu Lys Lys Gln Leu Ala Val Ala Glu Gly Lys Pro Pro Glu
   20 25 30
   Ala Pro Lys Gly Lys Lys Lys Lys
   35 40
<210> 3
   <211> 400
   <212> PRT
   <213> Artificial Sequence
<220>
   <223> Amino acid sequence of CK19(Cytokeratin 19)
<400> 3
   Met Thr Ser Tyr Ser Tyr Arg Gln Ser Ser Ala Thr Ser Ser Phe Gly
   1 5 10 15
   Gly Leu Gly Gly Gly Ser Val Arg Phe Gly Pro Gly Val Ala Phe Arg
   20 25 30
   Ala Pro Ser Ile His Gly Gly Ser Gly Gly Arg Gly Val Ser Val Ser
   35 40 45
   Ser Ala Arg Phe Val Ser Ser Ser Ser Ser Gly Ala Tyr Gly Gly Gly
   50 55 60
   Tyr Gly Gly Val Leu Thr Ala Ser Asp Gly Leu Leu Ala Gly Asn Glu
   65 70 75 80
   Lys Leu Thr Met Gln Asn Leu Asn Asp Arg Leu Ala Ser Tyr Leu Asp
   85 90 95
   Lys Val Arg Ala Leu Glu Ala Ala Asn Gly Glu Leu Glu Val Lys Ile
   100 105 110
   Arg Asp Trp Tyr Gln Lys Gln Gly Pro Gly Pro Ser Arg Asp Tyr Ser
   115 120 125
   His Tyr Tyr Thr Thr Ile Gln Asp Leu Arg Asp Lys Ile Leu Gly Ala
   130 135 140
   Thr Ile Glu Asn Ser Arg Ile Val Leu Gln Ile Asp Asn Ala Arg Leu
   145 150 155 160
   Ala Ala Asp Asp Phe Arg Thr Lys Phe Glu Thr Glu Gln Ala Leu Arg
   165 170 175
   Met Ser Val Glu Ala Asp Ile Asn Gly Leu Arg Arg Val Leu Asp Glu
   180 185 190
   Leu Thr Leu Ala Arg Thr Asp Leu Glu Met Gln Ile Glu Gly Leu Lys
   195 200 205
   Glu Glu Leu Ala Tyr Leu Lys Lys Asn His Glu Glu Glu Ile Ser Thr
   210 215 220
   Leu Arg Gly Gln Val Gly Gly Gln Val Ser Val Glu Val Asp Ser Ala
   225 230 235 240
   Pro Gly Thr Asp Leu Ala Lys Ile Leu Ser Asp Met Arg Ser Gln Tyr
   245 250 255
   Glu Val Met Ala Glu Gln Asn Arg Lys Asp Ala Glu Ala Trp Phe Thr
   260 265 270
   Ser Arg Thr Glu Glu Leu Asn Arg Glu Val Ala Gly His Thr Glu Gln
   275 280 285
   Leu Gln Met Ser Arg Ser Glu Val Thr Asp Leu Arg Arg Thr Leu Gln
   290 295 300
   Gly Leu Glu Ile Glu Leu Gln Ser Gln Leu Ser Met Lys Ala Ala Leu
   305 310 315 320
   Glu Asp Thr Leu Ala Glu Thr Glu Ala Arg Phe Gly Ala Gln Leu Ala
   325 330 335
   His Ile Gln Ala Leu Ile Ser Gly Ile Glu Ala Gln Leu Gly Asp Val
   340 345 350
   Arg Ala Asp Ser Glu Arg Gln Asn Gln Glu Tyr Gln Arg Leu Met Asp
   355 360 365
   Ile Lys Ser Arg Leu Glu Gln Glu Ile Ala Thr Tyr Arg Ser Leu Leu
   370 375 380
   Glu Gly Gln Glu Asp His Tyr Asn Asn Leu Ser Ala Ser Lys Val Leu
   385 390 395 400
<210> 4
   <211> 17
   <212> PRT
   <213> Artificial Sequence
<220>
   <223> 1E8 VL CDR1
<400> 4
   Lys Ser Ser Gln Ser Leu Leu Tyr Ser Ser Asn Gln Lys Asn Tyr Leu
   1 5 10 15
   Ala
<210> 5
   <211> 51
   <212> DNA
   <213> Artificial Sequence
<220>
   <223> 1E8 VL CDR1
<400> 5
   aagtccagtc agagcctttt atatagtagc aatcaaaaga actacttggc c 51
<210> 6
   <211> 7
   <212> PRT
   <213> Artificial Sequence
<220>
   <223> 1E8 VL CDR2
<400> 6
   Trp Ala Ser Thr Arg Glu Ser
   1 5
<210> 7
   <211> 21
   <212> DNA
   <213> Artificial Sequence
<220>
   <223> 1E8 VL CDR2
<400> 7
   tgggcatcca ctagggaatc t 21
<210> 8
   <211> 8
   <212> PRT
   <213> Artificial Sequence
<220>
   <223> 1E8 VL CDR3
<400> 8
   Gln Gln Tyr Tyr Ser Tyr Pro Thr
   1 5
<210> 9
   <211> 24
   <212> DNA
   <213> Artificial Sequence
<220>
   <223> 1E8 VL CDR3
<400> 9
   cagcaatatt atagctatcc gacg 24
<210> 10
   <211> 6
   <212> PRT
   <213> Artificial Sequence
<220>
   <223> 1E8 VH CDR1
<400> 10
   Ser Asp Tyr Ala Trp Asn
   1 5
<210> 11
   <211> 18
   <212> DNA
   <213> Artificial Sequence
<220>
   <223> 1E8 VH CDR1
<400> 11
   agtgattatg cctggaac 18
<210> 12
   <211> 16
   <212> PRT
   <213> Artificial Sequence
<220>
   <223> 1E8 VH CDR2
<400> 12
   Tyr Ile Ser Tyr Ser Gly Arg Thr Ser Tyr Lys Ser Ser Leu Lys Ser
   1 5 10 15
<210> 13
   <211> 48
   <212> DNA
   <213> Artificial Sequence
<220>
   <223> 1E8 VH CDR2
<400> 13
   tacataagct acagtggtcg cactagctac aaatcatctc tcaaaagt 48
<210> 14
   <211> 11
   <212> PRT
   <213> Artificial Sequence
<220>
   <223> 1E8 VH CDR3
<400> 14
   Asp Tyr Gly Asn Phe Val Gly Tyr Phe Asp Val
   1 5 10
<210> 15
   <211> 33
   <212> DNA
   <213> Artificial Sequence
<220>
   <223> 1E8 VH CDR3
<400> 15
   gactatggta acttcgtagg ttacttcgat gtc 33
<210> 16
   <211> 11
   <212> PRT
   <213> Artificial Sequence
<220>
   <223> 8A12 VL CDR1
<400> 16
   Lys Ala Ser Gln Asp Ile Asn Ser Tyr Leu Ser
   1 5 10
<210> 17
   <211> 33
   <212> DNA
   <213> Artificial Sequence
<220>
   <223> 8A12 VL CDR1
<400> 17
   aaggcgagtc aggacattaa tagctattta agc 33
<210> 18
   <211> 7
   <212> PRT
   <213> Artificial Sequence
<220>
   <223> 8A12 VL CDR2
<400> 18
   Arg Ala Asn Arg Leu Val Asp
   1 5
<210> 19
   <211> 21
   <212> DNA
   <213> Artificial Sequence
<220>
   <223> 8A12 VL CDR2
<400> 19
   cgtgcaaaca gattggtaga t 21
<210> 20
   <211> 9
   <212> PRT
   <213> Artificial Sequence
<220>
   <223> 8A12 VL CDR3
<400> 20
   Leu Gln Tyr Asp Glu Phe Pro Arg Thr
   1 5
<210> 21
   <211> 27
   <212> DNA
   <213> Artificial Sequence
<220>
   <223> 8A12 VL CDR3
<400> 21
   ctacagtatg atgagtttcc tcggacg 27
<210> 22
   <211> 6
   <212> PRT
   <213> Artificial Sequence
<220>
   <223> 8A12 VH CDR1
<400> 22
   Ser Glu Tyr Ala Trp Thr
   1 5
<210> 23
   <211> 18
   <212> DNA
   <213> Artificial Sequence
<220>
   <223> 8A12 VH CDR1
<400> 23
   agtgagtatg cctggacc 18
<210> 24
   <211> 16
   <212> PRT
   <213> Artificial Sequence
<220>
   <223> 8A12 VH CDR2
<400> 24
   Tyr Ile Asn Tyr Asn Gly Asn Thr Asn Leu Asn Pro Ser Leu Lys Ser
   1 5 10 15
<210> 25
   <211> 48
   <212> DNA
   <213> Artificial Sequence
<220>
   <223> 8A12 VH CDR2
<400> 25
   tacataaact acaatggcaa cactaactta aatccatctc tcaaaagt 48
<210> 26
   <211> 10
   <212> PRT
   <213> Artificial Sequence
<220>
   <223> 8A12 VH CDR3
<400> 26
   Ser Leu Trp Pro Arg Gly Trp Phe Ala Tyr
   1 5 10
<210> 27
   <211> 30
   <212> DNA
   <213> Artificial Sequence
<220>
   <223> 8A12 VH CDR3
<400> 27
   tcactttggc ccaggggctg gtttgcttac 30
<210> 28
   <211> 112
   <212> PRT
   <213> Artificial Sequence
<220>
   <223> 1E8 VL
<400> 28
   Asp Ile Val Met Thr Gln Ser Pro Ser Ser Leu Ala Val Ser Val Gly
   1 5 10 15
   Glu Lys Val Thr Met Ser Cys Lys Ser Ser Gln Ser Leu Leu Tyr Ser
   20 25 30
   Ser Asn Gln Lys Asn Tyr Leu Ala Trp Tyr Gln Gln Lys Pro Gly Gln
   35 40 45
   Ser Pro Lys Leu Leu Ile Tyr Trp Ala Ser Thr Arg Glu Ser Gly Val
   50 55 60
   Pro Asp Arg Phe Thr Gly Ser Gly Ser Gly Thr Glu Phe Thr Leu Thr
   65 70 75 80
   Ile Ser Ser Val Lys Ala Glu Asp Leu Ala Val Tyr Tyr Cys Gln Gln
   85 90 95
   Tyr Tyr Ser Tyr Pro Thr Phe Gly Gly Gly Thr Lys Leu Glu Ile Lys
   100 105 110
<210> 29
   <211> 336
   <212> DNA
   <213> Artificial Sequence
<220>
   <223> 1E8 VL
<400> 29
   gacattgtga tgacccagtc tccatcctcc ctagctgtgt cagttggaga gaaggttact 60
   atgagctgca agtccagtca gagcctttta tatagtagca atcaaaagaa ctacttggcc 120
   tggtaccagc agaaaccagg gcagtctcct aaactgctga tttactgggc atccactagg 180
   gaatctgggg tccctgatcg cttcacaggc agtggatctg ggacagaatt cactctcacc 240
   atcagcagtg tgaaggctga agacctggca gtttattact gtcagcaata ttatagctat 300
   ccgacgttcg gtggaggcac caagctggaa atcaaa 336
<210> 30
   <211> 120
   <212> PRT
   <213> Artificial Sequence
<220>
   <223> 1E8 VH
<400> 30
   Asp Val Lys Leu Gln Glu Ser Gly Pro Gly Leu Val Asn Pro Ser Gln
   1               5                   10                  15
   Ser Leu Ser Leu Thr Cys Thr Val Thr Gly Tyr Ser Ile Thr Ser Asp
               20                  25                  30
   Tyr Ala Trp Asn Trp Ile Arg Gln Phe Pro Gly Asn Lys Leu Glu Trp
           35                  40                  45
   Met Gly Tyr Ile Ser Tyr Ser Gly Arg Thr Ser Tyr Lys Ser Ser Leu
       50                  55                  60
   Lys Ser Arg Ile Ser Ile Thr Arg Asp Thr Ser Lys Asn Gln Phe Phe
   65                  70                  75                  80
   Leu Glu Leu Asn Ser Val Thr Thr Glu Asp Thr Ala Thr Tyr Tyr Cys
                   85                  90                  95
   Ala Arg Asp Tyr Gly Asn Phe Val Gly Tyr Phe Asp Val Trp Gly Ala
               100                 105                 110
   Gly Thr Thr Val Thr Val Ser Ser
           115                 120
<210> 31
   <211> 360
   <212> DNA
   <213> Artificial Sequence
<220>
   <223> 1E8 VH
<400> 31 360
<210> 32
   <211> 107
   <212> PRT
   <213> Artificial Sequence
<220>
   <223> 8A12 VL
<400> 32
   Asp Ile Gln Met Thr Gln Ser Pro Ser Ser Met Tyr Ala Ser Leu Gly
   1               5                   10                  15
   Glu Arg Val Thr Ile Thr Cys Lys Ala Ser Gln Asp Ile Asn Ser Tyr
               20                  25                  30
   Leu Ser Trp Phe Gln Gln Lys Pro Gly Lys Ser Pro Lys Thr Leu Met
           35                  40                  45
   Tyr Arg Ala Asn Arg Leu Val Asp Gly Val Pro Ser Arg Phe Ser Gly
       50                  55                  60
   Ser Gly Ser Gly Gln Asp Tyr Ser Leu Thr Ile Ser Ser Leu Glu Tyr
   65                  70                  75                  80
   Glu Asp Met Gly Ile Tyr Tyr Cys Leu Gln Tyr Asp Glu Phe Pro Arg
                   85                  90                  95
   Thr Phe Gly Gly Gly Thr Lys Leu Glu Ile Lys
               100                 105
<210> 33
   <211> 321
   <212> DNA
   <213> Artificial Sequence
<220>
   <223> 8A12 VL
<400> 33
   gacattctga tgacccagtc tccatcttcc atgtatgcat ctctaggaga gagagtcact      60
   atcacttgca aggcgagtca ggacattaat agctatttaa gctggttcca gcagaaacca     120
   gggaaatctc ctaagaccct gatgtatcgt gcaaacagat tggtagatgg ggtcccatca     180
   aggttcagtg gcagtggatc tggccaagat tattctctca ccatcagcag cctggaatat     240
   gaagatatgg gaatttatta ttgtctacag tatgatgagt ttcctcggac gttcggtgga     300
   ggcaccaagc tggaaatcaa a                                                321
<210> 34
   <211> 119
   <212> PRT
   <213> Artificial Sequence
<220>
   <223> 8A12 VH
<400> 34
   Asp Val Lys Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr Ser Glu
   20 25 30
   Tyr Ala Trp Thr Trp Ile Arg Gln Phe Pro Gly Asn Lys Leu Glu Trp
   35 40 45
   Met Gly Tyr Ile Asn Tyr Asn Gly Asn Thr Asn Leu Asn Pro Ser Leu
   50 55 60
   Lys Ser Arg Ile Ser Ile Ile Arg Asp Thr Ser Lys Asn Gln Phe Phe
   65 70 75 80
   Leu Gln Leu Asn Ser Val Thr Thr Glu Asp Thr Ala Thr Tyr Tyr Cys
   85 90 95
   Ala Arg Ser Leu Trp Pro Arg Gly Trp Phe Ala Tyr Trp Gly Gln Gly
   100 105 110
   Thr Leu Val Thr Val Ser Ala
   115
<210> 35
   <211> 357
   <212> DNA
   <213> Artificial Sequence
<220>
   <223> 8A12 VH
<400> 35
   gatgtgaagc ttcaggagtc gggacctggc ctggtgaaac cttctcagtc tctgtccctc 60
   acctgcactg tcactggcta ttcaatcacc agtgagtatg cctggacctg gatccggcag 120
   tttccaggaa acaaactgga atggatgggc tacataaact acaatggcaa cactaactta 180
   aatccatctc tcaaaagtcg aatctctatc attcgagaca catccaagaa ccagttcttc 240
   ctgcagttga attctgtgac aactgaggac acagccacat attactgtgc aagatcactt 300
   tggcccaggg gctggtttgc ttactggggc caagggactc tggtcactgt ctctgca 357
<210> 36
   <211> 218
   <212> PRT
   <213> Artificial Sequence
<220>
   <223> 1E8 light chain
<400> 36
   Asp Ile Val Met Thr Gln Ser Pro Ser Ser Leu Ala Val Ser Val Gly
   1               5                   10                  15
   Glu Lys Val Thr Met Ser Cys Lys Ser Ser Gln Ser Leu Leu Tyr Ser
               20                  25                  30
   Ser Asn Gln Lys Asn Tyr Leu Ala Trp Tyr Gln Gln Lys Pro Gly Gln
   35 40 45
   Ser Pro Lys Leu Leu Ile Tyr Trp Ala Ser Thr Arg Glu Ser Gly Val
   50 55 60
   Pro Asp Arg Phe Thr Gly Ser Gly Ser Gly Thr Glu Phe Thr Leu Thr
   65 70 75 80
   Ile Ser Ser Val Lys Ala Glu Asp Leu Ala Val Tyr Tyr Cys Gln Gln
   85 90 95
   Tyr Tyr Ser Tyr Pro Thr Phe Gly Gly Gly Thr Lys Leu Glu Ile Lys
   100 105 110
   Ala Asp Ala Ala Pro Thr Val Ser Ile Phe Pro Pro Ser Ser Glu Gln
   115 120 125
   Leu Thr Ser Gly Gly Ala Ser Val Val Cys Phe Leu Asn Asn Phe Tyr
   130 135 140
   Pro Lys Asp Ile Asn Val Lys Trp Lys Ile Asp Gly Ser Glu Arg Gln
   145 150 155 160
   Asn Gly Val Leu Asn Ser Trp Thr Asp Gln Asp Ser Lys Asp Ser Thr
   165 170 175
   Tyr Ser Met Ser Arg Thr Leu Thr Leu Thr Lys Asp Glu Tyr Glu Arg
   180 185 190
   His Asn Ser Tyr Thr Cys Glu Ala Thr His Lys Thr Ser Thr Ser Pro
   195 200 205
   Ile Val Lys Ser Phe Asn Arg Asn Glu Cys
   210 215
<210> 37
   <211> 450
   <212> PRT
   <213> Artificial Sequence
<220>
   <223> 1E8 Heavy chain
<400> 37
   Asp Val Lys Leu Gln Glu Ser Gly Pro Gly Leu Val Asn Pro Ser Gln
   1 5 10 15
   Ser Leu Ser Leu Thr Cys Thr Val Thr Gly Tyr Ser Ile Thr Ser Asp
   20 25 30
   Tyr Ala Trp Asn Trp Ile Arg Gln Phe Pro Gly Asn Lys Leu Glu Trp
   35 40 45
   Met Gly Tyr Ile Ser Tyr Ser Gly Arg Thr Ser Tyr Lys Ser Ser Leu
   50 55 60
   Lys Ser Arg Ile Ser Ile Thr Arg Asp Thr Ser Lys Asn Gln Phe Phe
   65 70 75 80
   Leu Glu Leu Asn Ser Val Thr Thr Glu Asp Thr Ala Thr Tyr Tyr Cys
   85 90 95
   Ala Arg Asp Tyr Gly Asn Phe Val Gly Tyr Phe Asp Val Trp Gly Ala
   100 105 110
   Gly Thr Thr Val Thr Val Ser Ser Ala Lys Thr Thr Ala Pro Ser Val
   115 120 125
   Tyr Pro Leu Ala Pro Val Cys Gly Asp Thr Thr Gly Ser Ser Val Thr
   130 135 140
   Leu Gly Cys Leu Val Lys Gly Tyr Phe Pro Glu Pro Val Thr Leu Thr
   145 150 155 160
   Trp Asn Ser Gly Ser Leu Ser Ser Gly Val His Thr Phe Pro Ala Val
   165 170 175
   Leu Gln Ser Asp Leu Tyr Thr Leu Ser Ser Ser Val Thr Val Thr Ser
   180 185 190
   Ser Thr Trp Pro Ser Gln Ser Ile Thr Cys Asn Val Ala His Pro Ala
   195 200 205
   Ser Ser Thr Lys Val Asp Lys Lys Ile Glu Pro Arg Gly Pro Thr Ile
   210 215 220
   Lys Pro Cys Pro Pro Cys Lys Cys Pro Ala Pro Asn Leu Leu Gly Gly
   225 230 235 240
   Pro Ser Val Phe Ile Phe Pro Pro Lys Ile Lys Asp Val Leu Met Ile
   245 250 255
   Ser Leu Ser Pro Ile Val Thr Cys Val Val Val Asp Val Ser Glu Asp
   260 265 270
   Asp Pro Asp Val Gln Ile Ser Trp Phe Val Asn Asn Val Glu Val His
   275 280 285
   Thr Ala Gln Thr Gln Thr His Arg Glu Asp Tyr Asn Ser Thr Leu Arg
   290 295 300
   Val Val Ser Ala Leu Pro Ile Gln His Gln Asp Trp Met Ser Gly Lys
   305 310 315 320
   Glu Phe Lys Cys Lys Val Asn Asn Lys Asp Leu Pro Ala Pro Ile Glu
   325 330 335
   Arg Thr Ile Ser Lys Pro Lys Gly Ser Val Arg Ala Pro Gln Val Tyr
   340 345 350
   Val Leu Pro Pro Pro Glu Glu Glu Met Thr Lys Lys Gln Val Thr Leu
   355 360 365
   Thr Cys Met Val Thr Asp Phe Met Pro Glu Asp Ile Tyr Val Glu Trp
   370 375 380
   Thr Asn Asn Gly Lys Thr Glu Leu Asn Tyr Lys Asn Thr Glu Pro Val
   385 390 395 400
   Leu Asp Ser Asp Gly Ser Tyr Phe Met Tyr Ser Lys Leu Arg Val Glu
   405 410 415
   Lys Lys Asn Trp Val Glu Arg Asn Ser Tyr Ser Cys Ser Val Val His
   420 425 430
   Glu Gly Leu His Asn His His Thr Thr Lys Ser Phe Ser Arg Thr Pro
   435 440 445
   Gly Lys
   450
<210> 38
   <211> 213
   <212> PRT
   <213> Artificial Sequence
<220>
   <223> 8A12 light chain
<400> 38
   Asp Ile Gln Met Thr Gln Ser Pro Ser Ser Met Tyr Ala Ser Leu Gly
   1 5 10 15
   Glu Arg Val Thr Ile Thr Cys Lys Ala Ser Gln Asp Ile Asn Ser Tyr
   20 25 30
   Leu Ser Trp Phe Gln Gln Lys Pro Gly Lys Ser Pro Lys Thr Leu Met
   35 40 45
   Tyr Arg Ala Asn Arg Leu Val Asp Gly Val Pro Ser Arg Phe Ser Gly
   50 55 60
   Ser Gly Ser Gly Gln Asp Tyr Ser Leu Thr Ile Ser Ser Leu Glu Tyr
   65 70 75 80
   Glu Asp Met Gly Ile Tyr Tyr Cys Leu Gln Tyr Asp Glu Phe Pro Arg
   85 90 95
   Thr Phe Gly Gly Gly Thr Lys Leu Glu Ile Lys Ala Asp Ala Ala Pro
   100 105 110
   Thr Val Ser Ile Phe Pro Pro Ser Ser Glu Gln Leu Thr Ser Gly Gly
   115 120 125
   Ala Ser Val Val Cys Phe Leu Asn Asn Phe Tyr Pro Lys Asp Ile Asn
   130 135 140
   Val Lys Trp Lys Ile Asp Gly Ser Glu Arg Gln Asn Gly Val Leu Asn
   145 150 155 160
   Ser Trp Thr Asp Gln Asp Ser Lys Asp Ser Thr Tyr Ser Met Ser Arg
   165 170 175
   Thr Leu Thr Leu Thr Lys Asp Glu Tyr Glu Arg His Asn Ser Tyr Thr
   180 185 190
   Cys Glu Ala Thr His Lys Thr Ser Thr Ser Pro Ile Val Lys Ser Phe
   195 200 205
   Asn Arg Asn Glu Cys
   210
<210> 39
   <211> 449
   <212> PRT
   <213> Artificial Sequence
<220>
   <223> 8A12 Heavy chain
<400> 39
   Asp Val Lys Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr Ser Glu
   20 25 30
   Tyr Ala Trp Thr Trp Ile Arg Gln Phe Pro Gly Asn Lys Leu Glu Trp
   35 40 45
   Met Gly Tyr Ile Asn Tyr Asn Gly Asn Thr Asn Leu Asn Pro Ser Leu
   50 55 60
   Lys Ser Arg Ile Ser Ile Ile Arg Asp Thr Ser Lys Asn Gln Phe Phe
   65 70 75 80
   Leu Gln Leu Asn Ser Val Thr Thr Glu Asp Thr Ala Thr Tyr Tyr Cys
   85 90 95
   Ala Arg Ser Leu Trp Pro Arg Gly Trp Phe Ala Tyr Trp Gly Gln Gly
   100 105 110
   Thr Leu Val Thr Val Ser Ala Ala Lys Thr Thr Ala Pro Ser Val Tyr
   115 120 125
   Pro Leu Ala Pro Val Cys Gly Asp Thr Thr Gly Ser Ser Val Thr Leu
   130 135 140
   Gly Cys Leu Val Lys Gly Tyr Phe Pro Glu Pro Val Thr Leu Thr Trp
   145 150 155 160
   Asn Ser Gly Ser Leu Ser Ser Gly Val His Thr Phe Pro Ala Val Leu
   165 170 175
   Gln Ser Asp Leu Tyr Thr Leu Ser Ser Ser Val Thr Val Thr Ser Ser
   180 185 190
   Thr Trp Pro Ser Gln Ser Ile Thr Cys Asn Val Ala His Pro Ala Ser
   195 200 205
   Ser Thr Lys Val Asp Lys Lys Ile Glu Pro Arg Gly Pro Thr Ile Lys
   210 215 220
   Pro Cys Pro Pro Cys Lys Cys Pro Ala Pro Asn Leu Leu Gly Gly Pro
   225 230 235 240
   Ser Val Phe Ile Phe Pro Pro Lys Ile Lys Asp Val Leu Met Ile Ser
   245 250 255
   Leu Ser Pro Ile Val Thr Cys Val Val Val Asp Val Ser Glu Asp Asp
   260 265 270
   Pro Asp Val Gln Ile Ser Trp Phe Val Asn Asn Val Glu Val His Thr
   275 280 285
   Ala Gln Thr Gln Thr His Arg Glu Asp Tyr Asn Ser Thr Leu Arg Val
   290 295 300
   Val Ser Ala Leu Pro Ile Gln His Gln Asp Trp Met Ser Gly Lys Glu
   305 310 315 320
   Phe Lys Cys Lys Val Asn Asn Lys Asp Leu Pro Ala Pro Ile Glu Arg
   325 330 335
   Thr Ile Ser Lys Pro Lys Gly Ser Val Arg Ala Pro Gln Val Tyr Val
   340 345 350
   Leu Pro Pro Pro Glu Glu Glu Met Thr Lys Lys Gln Val Thr Leu Thr
   355 360 365
   Cys Met Val Thr Asp Phe Met Pro Glu Asp Ile Tyr Val Glu Trp Thr
   370 375 380
   Asn Asn Gly Lys Thr Glu Leu Asn Tyr Lys Asn Thr Glu Pro Val Leu
   385 390 395 400
   Asp Ser Asp Gly Ser Tyr Phe Met Tyr Ser Lys Leu Arg Val Glu Lys
   405 410 415
   Lys Asn Trp Val Glu Arg Asn Ser Tyr Ser Cys Ser Val Val His Glu
   420 425 430
   Gly Leu His Asn His His Thr Thr Lys Ser Phe Ser Arg Thr Pro Gly
   435 440 445
   Lys
<210> 40
   <211> 174
   <212> PRT
   <213> Artificial Sequence
<220>
   <223> AIMP3(Aminoacyl tRNA synthetase complex-interacting multifunctional protein 3)
<400> 40
   Met Ala Ala Ala Ala Glu Leu Ser Leu Leu Glu Lys Ser Leu Gly Leu
   1               5                   10                  15
   Ser Lys Gly Asn Lys Tyr Ser Ala Gln Gly Glu Arg Gln Ile Pro Val
               20                  25                  30
   Leu Gln Thr Asn Asn Gly Pro Ser Leu Thr Gly Leu Thr Thr Ile Ala
           35                  40                  45
   Ala His Leu Val Lys Gln Ala Asn Lys Glu Tyr Leu Leu Gly Ser Thr
       50                  55                  60
   Ala Glu Glu Lys Ala Ile Val Gln Gln Trp Leu Glu Tyr Arg Val Thr
   65                  70                  75                  80
   Gln Val Asp Gly His Ser Ser Lys Asn Asp Ile His Thr Leu Leu Lys
                   85                  90                  95
   Asp Leu Asn Ser Tyr Leu Glu Asp Lys Val Tyr Leu Thr Gly Tyr Asn
               100                 105                 110
   Phe Thr Leu Ala Asp Ile Leu Leu Tyr Tyr Gly Leu His Arg Phe Ile
           115                 120                 125
   Val Asp Leu Thr Val Gln Glu Lys Glu Lys Tyr Leu Asn Val Ser Arg
       130                 135                 140
   Trp Phe Cys His Ile Gln His Tyr Pro Gly Ile Arg Gln His Leu Ser
   145                 150                 155                 160
   Ser Val Val Phe Ile Lys Asn Arg Leu Tyr Thr Asn Ser His
                   165                 170
<210> 41
   <211> 30
   <212> PRT
   <213> Artificial Sequence
<220>
   <223> 1E8 VH FR1
<400> 41
   Asp Val Lys Leu Gln Glu Ser Gly Pro Gly Leu Val Asn Pro Ser Gln
   1 5 10 15
   Ser Leu Ser Leu Thr Cys Thr Val Thr Gly Tyr Ser Ile Thr
   20 25 30
<210> 42
   <211> 90
   <212> DNA
   <213> Artificial Sequence
<220>
   <223> 1E8 VH FR1
<400> 42
   gatgtgaagc ttcaggagtc gggacctggc ctggtgaatc cttctcagtc tctgtccctc 60
   acctgcactg tcactggcta ttcaatcacc 90
<210> 43
   <211> 14
   <212> PRT
   <213> Artificial Sequence
<220>
   <223> 1E8 VH FR2
<400> 43
   Trp Ile Arg Gln Phe Pro Gly Asn Lys Leu Glu Trp Met Gly
   1 5 10
<210> 44
   <211> 42
   <212> DNA
   <213> Artificial Sequence
<220>
   <223> 1E8 VH FR2
<400> 44
   tggatccggc agtttccagg aaacaaactg gagtggatgg gc 42
<210> 45
   <211> 32
   <212> PRT
   <213> Artificial Sequence
<220>
   <223> 1E8 VH FR3
<400> 45
   Arg Ile Ser Ile Thr Arg Asp Thr Ser Lys Asn Gln Phe Phe Leu Glu
   1 5 10 15
   Leu Asn Ser Val Thr Thr Glu Asp Thr Ala Thr Tyr Tyr Cys Ala Arg
   20 25 30
<210> 46
   <211> 96
   <212> DNA
   <213> Artificial Sequence
<220>
   <223> 1E8 VH FR3
<400> 46
   cgaatctcta tcactcgaga cacatccaag aaccagttct tcctggagtt gaattctgtg 60
   actactgagg acacagccac atattactgt gcaaga 96
<210> 47
   <211> 11
   <212> PRT
   <213> Artificial Sequence
<220>
   <223> 1E8 VH FR4
<400> 47
   Trp Gly Ala Gly Thr Thr Val Thr Val Ser Ser
   1 5 10
<210> 48
   <211> 33
   <212> DNA
   <213> Artificial Sequence
<220>
   <223> 1E8 VH FR4
<400> 48
   tggggcgcag ggaccacggt caccgtctcc tca 33
<210> 49
   <211> 23
   <212> PRT
   <213> Artificial Sequence
<220>
   <223> 1E8 VL FR1
<400> 49
   Asp Ile Val Met Thr Gln Ser Pro Ser Ser Leu Ala Val Ser Val Gly
   1               5                   10                  15
   Glu Lys Val Thr Met Ser Cys
               20
<210> 50
   <211> 69
   <212> DNA
   <213> Artificial Sequence
<220>
   <223> 1E8 VL FR1
<400> 50
   gacattgtga tgacccagtc tccatcctcc ctagctgtgt cagttggaga gaaggttact      60
   atgagctgc                                                              69
<210> 51
   <211> 15
   <212> PRT
   <213> Artificial Sequence
<220>
   <223> 1E8 VL FR2
<400> 51
   Trp Tyr Gln Gln Lys Pro Gly Gln Ser Pro Lys Leu Leu Ile Tyr
   1               5                   10                  15
<210> 52
   <211> 45
   <212> DNA
   <213> Artificial Sequence
<220>
   <223> 1E8 VL FR2
<400> 52
   tggtaccagc agaaaccagg gcagtctcct aaactgctga tttac 45
<210> 53
   <211> 32
   <212> PRT
   <213> Artificial Sequence
<220>
   <223> 1E8 VL FR3
<400> 53
   Gly Val Pro Asp Arg Phe Thr Gly Ser Gly Ser Gly Thr Glu Phe Thr
   1 5 10 15
   Leu Thr Ile Ser Ser Val Lys Ala Glu Asp Leu Ala Val Tyr Tyr Cys
   20 25 30
<210> 54
   <211> 96
   <212> DNA
   <213> Artificial Sequence
<220>
   <223> 1E8 VL FR3
<400> 54
   ggggtccctg atcgcttcac aggcagtgga tctgggacag aattcactct caccatcagc 60
   agtgtgaagg ctgaagacct ggcagtttat tactgt 96
<210> 55
   <211> 10
   <212> PRT
   <213> Artificial Sequence
<220>
   <223> 1E8 VL FR4
<400> 55
   Phe Gly Gly Gly Thr Lys Leu Glu Ile Lys
   1 5 10
<210> 56
   <211> 30
   <212> DNA
   <213> Artificial Sequence
<220>
   <223> 1E8 VL FR4
<400> 56
   ttcggtggag gcaccaagct ggaaatcaaa 30
<210> 57
   <211> 30
   <212> PRT
   <213> Artificial Sequence
<220>
   <223> 8A12 VH FR1
<400> 57
   Asp Val Lys Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr
   20 25 30
<210> 58
   <211> 90
   <212> DNA
   <213> Artificial Sequence
<220>
   <223> 8A12 VH FR1
<400> 58
   gatgtgaagc ttcaggagtc gggacctggc ctggtgaaac cttctcagtc tctgtccctc 60
   acctgcactg tcactggcta ttcaatcacc 90
<210> 59
   <211> 14
   <212> PRT
   <213> Artificial Sequence
<220>
   <223> 8A12 VH FR2
<400> 59
   Trp Ile Arg Gln Phe Pro Gly Asn Lys Leu Glu Trp Met Gly
   1 5 10
<210> 60
   <211> 42
   <212> DNA
   <213> Artificial Sequence
<220>
   <223> 8A12 VH FR2
<400> 60
   tggatccggc agtttccagg aaacaaactg gaatggatgg gc 42
<210> 61
   <211> 32
   <212> PRT
   <213> Artificial Sequence
<220>
   <223> 8A12 VH FR3
<400> 61
   Arg Ile Ser Ile Ile Arg Asp Thr Ser Lys Asn Gln Phe Phe Leu Gln
   1 5 10 15
   Leu Asn Ser Val Thr Thr Glu Asp Thr Ala Thr Tyr Tyr Cys Ala Arg
   20 25 30
<210> 62
   <211> 96
   <212> DNA
   <213> Artificial Sequence
<220>
   <223> 8A12 VH FR3
<400> 62
   cgaatctcta tcattcgaga cacatccaag aaccagttct tcctgcagtt gaattctgtg 60
   acaactgagg acacagccac atattactgt gcaaga 96
<210> 63
   <211> 11
   <212> PRT
   <213> Artificial Sequence
<220>
   <223> 8A12 VH FR4
<400> 63
   Trp Gly Gln Gly Thr Leu Val Thr Val Ser Ala
   1 5 10
<210> 64
   <211> 33
   <212> DNA
   <213> Artificial Sequence
<220>
   <223> 8A12 VH FR4
<400> 64
   tggggccaag ggactctggt cactgtctct gca 33
<210> 65
   <211> 23
   <212> PRT
   <213> Artificial Sequence
<220>
   <223> 8A12 VL FR1
<400> 65
   Asp Ile Gln Met Thr Gln Ser Pro Ser Ser Met Tyr Ala Ser Leu Gly
   1               5                   10                  15
   Glu Arg Val Thr Ile Thr Cys
               20
<210> 66
   <211> 69
   <212> DNA
   <213> Artificial Sequence
<220>
   <223> 8A12 VL FR1
<400> 66
   gacattctga tgacccagtc tccatcttcc atgtatgcat ctctaggaga gagagtcact        60
   atcacttgc                                                                 69
<210> 67
   <211> 15
   <212> PRT
   <213> Artificial Sequence
<220>
   <223> 8A12 VL FR2
<400> 67
   Trp Phe Gln Gln Lys Pro Gly Lys Ser Pro Lys Thr Leu Met Tyr
   1               5                   10                  15
<210> 68
   <211> 45
   <212> DNA
   <213> Artificial Sequence
<220>
   <223> 8A12 VL FR2
<400> 68
   tggttccagc agaaaccagg gaaatctcct aagaccctga tgtat 45
<210> 69
   <211> 32
   <212> PRT
   <213> Artificial Sequence
<220>
   <223> 8A12 VL FR3
<400> 69
   Gly Val Pro Ser Arg Phe Ser Gly Ser Gly Ser Gly Gln Asp Tyr Ser
   1               5                   10                  15
   Leu Thr Ile Ser Ser Leu Glu Tyr Glu Asp Met Gly Ile Tyr Tyr Cys
               20                  25                  30
<210> 70
   <211> 96
   <212> DNA
   <213> Artificial Sequence
<220>
   <223> 8A12 VL FR3
<400> 70
   ggggtcccat caaggttcag tggcagtgga tctggccaag attattctct caccatcagc        60
   agcctggaat atgaagatat gggaatttat tattgt                                  96
<210> 71
   <211> 10
   <212> PRT
   <213> Artificial Sequence
<220>
   <223> 8A12 VL FR4
<400> 71
   Phe Gly Gly Gly Thr Lys Leu Glu Ile Lys
   1               5                   10
<210> 72
   <211> 30
   <212> DNA
   <213> Artificial Sequence
<220>
   <223> 8A12 VL FR4
<400> 72
   ttcggtggag gcaccaagct ggaaatcaaa 30